# EUROPEAN PATENT SPECIFICATION

(11) **EP 0 502 976 B1**
(45) Date of publication and mention of the grant of the patent: **03.07.1996**
(21) Application number: 91901026.4
(22) Date of filing: 30.11.1990
(51) Int. Cl.: C12N 15/00, A01K 67/027, C07H 21/00, A23C 9/00

(54) **PRODUCTION OF RECOMBINANT POLYPEPTIDES BY BOVINE SPECIES AND TRANSGENIC METHODS**
HERSTELLUNG REKOMBINANTER POLYPEPTIDE DURCH RINDER UND TRANSGENE METHODEN
PRODUCTION DE POLYPEPTIDES RECOMBINES PAR DES ESPECES BOVINES ET PROCEDES TRANSGENIQUES

(30) Priority: 01.12.1989 US 444745
(43) Date of publication of application: 16.09.1992
(60) Divisional application: 95203326.4
(73) Proprietor: PHARMING B.V., 2333 CA Leiden (NL)
(72) Inventor: HEYNEKER, Herbert, L., Hillsborough, CA 94010 (US); DEBOER, Herman, A., NL-2371 GD Roelofarendsveen (NL); STRIJKER, Rein, NL-2352 CP Leiderdorp (NL); PLANTENBURG, Gerard, NL-2253 XS Voorschoten (NL); LEE, Sang, He, NL-2371 GN Roelofarendsveen (NL)
(74) Representative: Bizley, Richard Edward
(86) International application number: US9006874
(87) International publication number: WO9108216

(56) References cited:
- WO-A-88/01648
- WO-A-89/05864
- WO-A-90/05188
- WO-A-92/22647
- WO-A-92/22670
- US-A- 4 873 316
- BIO/TECHNOLOGY, vol. 7, May 1989; CLARK et al., pp. 487-492
- BIO/TECHNOLOGY, vol. 6, no. 10, October 1988; VAN BRUNT, pp. 1149-1154
- BIO/TECHNOLOGY, vol. 6, February 1988; SIMONS et al., pp. 179-183
- BIO/TECHNOLOGY, vol. 8, February 1990; BUHLER et al., pp. 140-143
- BIO/TECHNOLOGY, vol. 5, November 1987; GORDON et al., pp. 1183-1187
- THERIOGENOLOGY, vol. 29, no. 1, January 1988; BIERY et al., p. 224
- THERIOGENOLOGY, vol. 25, no. 1, January 1986; LOSKULOFF et al., p. 168
- THE NEW YORK TIMES NATIONAL, 08 June 1990; SCHNEIDER, "Texas researchers develop 4 gene-altered calves"
- PROCEEDINGS OF THE NATL. ACADEMY OF SCIENCES USA, vol. 83, July 1986, Washington, DC (US); JOHNSON et al., pp. 4660-4664
- JOURNAL OF DAIRY SCIENCE, vol. 72, October 1989; BREMEL et al., pp. 2826-2833
- BIOCHEMICAL & BIOPHYSICAL RESEARCH COMMUNICATIONS, vol. 135, no. 3, 28 March 1986; LEE et al., pp. 942-949

## Description

### Field of the Invention

The invention relates to the production of transgenic bovine species and to methods for producing transgenic non-human mammals having a desired phenotype.

### Background of the Invention

There is a plethora of literature relating to the expression of heterologous genes in lower organisms such as unicellular bacteria, yeast and filamentous fungi, and in higher cell types such as mammalian cells. There are also numerous reports on the production of transgenic animals, most of which relate to the production of transgenic mice. See, e.g. U.S. Pat. No. 4,736,866 (transgenic mice containing activated oncogene); Andres, A., et al. (1987) Proc. Natl. Acad. Sci. USA 84, 1299-1303 (HA-RAS oncogene under control of whey acid protein promoter); Schoenberger, C.A., et al. (1987) Experientia 43, 644 and (1988) EMBO J. 7. 169-175 (C-myc oncogene under control of whey acid protein promoter); and Muller, W.J., et al. (1988) Cell 54, 105-115 (C-myc oncogene under control of the mouse mammary tumor virus promoter). Several laboratories have also reported the production of transgenic porcine species (Miller, K.F., et al. (1989), J. Endocrin., 120, 481-488 (expression of human or bovine growth hormone gene in transgenic swine); Vize, P.D., et al. (1988), J. Cell Sci., 90, 295-300 (porcine growth hormone fusion gene in transgenic pigs); and Ebert, K. et al. (1988), Mol. Endocrin., 2, 277-283 (MMLV-rat somatotropin fusion gene in transgenic pigs)), transgenic sheep (Nancarrow, et al. (1987), Theriogenology, 27, 263 (transgenic sheep containing bovine growth hormone gene) Clark, A.J. et al. (1989) Bio/Technology 7, 487-482 and Simons, J., et al. (1988) Bio/Technology 6, 179-183 (human factor IX and α-1 antitrypsin CONA in ovine species), and rabbit (Hanover, S.V., et al. (1987), Deutche Tierarztliche Wochenschrift, 94, 476-478 (production of transgenic rabbits by injection of uteroglobin-promoter-CAT fusion gene into fertilized rabbit oocytes). A number of reports have also suggested the production of transgenic cattle (Wagner, et al. (1984), Theriogenology, 21, 29-44) with one reporting some progress in microinjection techniques (Lohse, J.K., et al. (1985), Theriogenology, 23, 205). However, little, if any, success has been achieved in producing transgenic cows. Scientific articles which clearly demonstrate the actual production of a transgenic cow capable of producing a heterologous protein are presently unknown. This, despite the statements that one transgenic cow was produced in Canada which expressed human β-interferon (Van Brunt, J. (1988), Bio/Technology, 6, 1149-1155) and that transient expression of human α-fetoprotein in liver and blood was obtained on one occasion (Church, R.B. (1986), Biotechnology News Watch, 6 (15), 4). One reference reports that bovine papilloma virus was apparently integrated but not expressed in a transgenic cow (Roschlau et al. (1988) Arch. Tierz., Berlin 31, 3-8). A recent article has summarized the genetic engineering of livestock. (Pursel, V.G. et al. (1989), Science, 244, 1281-1288).

A number of laboratories have reported tissue-specific expression of DNA encoding various proteins in the mammary gland or the production of various proteins in the milk of transgenic mice and sheep. For example, Simmons, J.P., et al. (1987) Nature 328, 530-532 report the microinjection of a 16.2kb genomic fragment encoding β-lactoglobulin (BLG) including 4kb of 5' sequence, 4.9kb of the BLG transcription unit and 7.3kb of 3' flanking sequence into fertilized mouse eggs. According to these authors, the sheep BLG was expressed in mammary tissue and produced BLG in the milk of the transgenic mice at concentrations ranging from about 3.0 to about 23 mg/ml. When, however, cDNA encoding human factor IX or human α1-antitrypsin was inserted into the 5' untranslated region of the BLG gene and microinjected into sheep (Simmons, J.P., et al. (1988) Bio/Technology 6, 179-183) the production of factor IX or al-antitrypsin was significantly reduced (25ng/ml for factor IX and 10mg/ml for α1-antitrypsin; see Clark, A.J., et al. (1989) Bio/Technology 7, 487-492).

In a similar approach, a 14kb genomic clone containing the entire 7.5kb rat β-casein together with 3.5kb of 5' and 3.0kb of 3' flanking DNA was reportedly microinjected into fertilized mouse oocytes. Lee, et al. (1988) Nucl. Acids Res. 16 1027-1041. Yet, in this case, the level of expression of the rat β-transgene in the lactating mammary gland of transgenic mice was reported to be at a level of 0.01-1% of the endogenous mouse β-casein gene.

Human tissue plasminogen activator (t-PA) reportedly was produced in transgenic mouse milk at the levels between 0.2 and about 0.4µg/ml when a cDNA encoding a human t-PA with its endogenous secretion sequence was expressed under control of a 2.6kb 5' sequence of the murine whey acid protein gene. Gordon, K., et al. (1987) Bio/Technology 5, 1183-1187. Subsequent experiments using the same or similar construction reportedly produced t-PA in different mouse lines arranging from less than 20ng of t-PA per ml of milk to about 50µg/ml. Pittius, C.W., et al. (1988) Proc. Natl. Acad. Sci. USA 85, 5874-5878.

U.S. Patent No. 4,873,316 issued October 10, 1989, discloses the use of 9kb of 5' sequence from the bovine αS1-casein gene including the casein signal peptide and several casein codons fused to a mature t-PA sequence. The transgenic mice obtained with this construct reportedly produced about 0.2-0.5µg/ml of a t-PA fusion protein in their milk.

In addition, a number of patent publications purportedly describe the production of specific proteins in the milk of transgenic mice and sheep. See, e.g. European Patent Publication No. 0 264 166 published April 20, 1988 (hepatitis B surface antigen and t-PA genes under control of the whey acid promoter protein for mammary tissue specific expression in mice); PCT Publication No. WO88/00239 published January 14, 1988 (tissue specific expression of a transgene encoding factor IX under control of a whey protein promoter in sheep); PCT Publication No. W088/01648 published March 10, 1988 (transgenic mouse having mammary secretory cells incorporating a recombinant expression system comprising a bovine α-lactalbumin gene fused to interleukin-2); European Pat. Pub. No. 0 279 582 published August 24, 1988 (tissue-specific expression of chloramphenicol acetyltransferase under control of rat β-casein promoter in transgenic mice); and PCT Pub. No. W088/10118 published December 29, 1988 (transgenic mice and sheep containing transgene encoding bovine αS1-casein promoter and signal sequence fused to t-PA).

Given the state of the transgenic art, it is apparent that a need exists for methods which enable the efficient production of transgenic bovines.

Further, it is apparent that a need exists for methods for producing transgenic bovine species which are capable of producing recombinant polypeptides such as human milk proteins and human serum proteins in the milk of such transgenic mammals.

### Summary of the Invention

Accordingly, the invention provides the use of in vitro maturation of a bovine ovum in the production of a transgenic bovine species of desired phenotype.

In another aspect the invention provides a method for producing an embryo of a transgenic bovine species which is capable of producing a recombinant polypeptide in at least one cell type, comprising:
obtaining an ovum from bovine ovaries;
obtaining maturation of the ovum in vitro and fertilizing to form a zygote;
introducing, optionally by microinjection, a transgene into the zygote; and
forming an embryo from the zygote.

In a further aspect the invention provides a method of producing a transgenic bovine species comprising transplanting an embryo which has been produced by a method as defined above into a recipient female bovine parent to permit the embryo to gestate and said parent to give birth.

It is an object herein to provide transgenic bovine species which are capable of producing recombinant polypeptides which are maintained intracellularly or are secreted extracellularly.

It is also an object herein to provide transgenic bovine species which are capable of producing recombinant polypeptides such as human milk proteins and human serum proteins in the milk of such transgenic animals.

Further, it is an object herein to provide milk from a transgenic bovine species containing such recombinant polypeptides.

Thus a further aspect of the invention is a process for producing bovine milk containing a recombinant polypeptide, which comprises:
obtaining an oocyte from bovine ovaries; obtaining maturation of the oocyte in vitro and fertilizing the oocyte to form a zygote;
introducing, optionally by microinjection, a transgene into the zygote;
forming an embryo from the zygote;
transplanting said embryo into a recipient female bovine parent to permit the embryo to gestate and said parent to give birth; and
obtaining milk from said transgenic bovine species.

The invention also includes a process for producing bovine milk containing a recombinant polypeptide, which comprises obtaining milk from a transgenic bovine species which has been produced by a method as described above.

The invention further includes a process for producing a recombinant polypeptide, which comprises:
obtaining an oocyte from bovine ovaries;
obtaining maturation of the oocyte in vitro and fertilizing the oocyte to form a zygote;
introducing, optionally by microinjection, a transgene into the zygote;
forming an embryo from the zygote;
transplanting said embryo into a recipient female bovine parent to permit the embryo to gestate and said parent to give birth;
obtaining milk from said transgenic bovine species; and
recovering said recombinant polypeptide from said milk.

The invention further includes a process for producing a recombinant polypeptide, which comprises recovering said polypeptide from milk which has been produced by a process as described above.

The invention further inclues a process for producing a pharmaceutical, which comprises:
obtaining an oocyte from bovine ovaries;
obtaining maturation of the oocyte in vitro and fertilizing the oocyte to form a zygote;
introducing, optionally by microinjection, a transgene into the zygote;
forming an embryo from the zygote;
transplanting said embryo into a recipient female bovine parent to permit the embryo to gestate and said parent to give birth;
obtaining milk from said transgenic bovine species;
recovering said recombinant polypeptide from said milk; and
using said recombinant polypeptide in making a pharmaceutical.

The invention further includes a process for producing a pharmaceutical, which comprises using a recombinant polypeptide which has been produced by a process as described above to make a pharmaceutical. application. Nothing herein is to be construed as an admission that the inventors are not entitled to antedate such disclosure by priority based on earlier filed applications.

The invention uses transgenes, inter alia, for producing recombinant polypeptides in the milk of transgenic bovine species. The production of such transgenic bovine milk containing one or more recombinant polypeptides is desirable since it provides a matrix wherein little or no purification is necessary for human consumption. The transgene comprises a secretory DNA sequence encoding a secretory signal sequence which is functional in mammary secretory cells of the bovine species of interest and a recombinant DNA sequence encoding the recombinant polypeptide. These sequences are operably linked to form a secretory-recombinant DNA sequence. At least one expression regulation sequence, functional in the mammary secretory cells of the bovine species, is operably linked to the secretory-recombinant DNA sequence. The transgene so constructed is capable of directing the expression of the secretory-recombinant DNA sequence in mammary secretory cells of bovine species containing the transgene. Such expression produces a form of recombinant polypeptide which is secreted from the mammary secretory cells into the milk of the transgenic bovine species.

The methods for producing such transgenic bovine species include introducing the above transgene into an embryonal target cell of a bovine species, transplanting the transgenic embryonic target cell formed thereby into a recipient bovine parent and identifying at least one female offspring which is capable of producing the recombinant polypeptide in its milk.

The invention thus enables the provision of transgenic bovine species capable of producing recombinant polypeptides in the milk of lactating females of said species. The milk from such transgenic bovine species containing such recombinant polypeptides can be used in food formulations containing the transgenic milk in liquid or dried form, as well as to provide food formulations supplemented with one or more recombinant polypeptides from such transgenic milk.

In addition to the foregoing, the invention enables the provision of transgenic bovine species containing transgenes that are capable of producing a recombinant polypeptide. Such transgenes are similar to the aforementioned transgenes for milk secretion and are characterized by having an expression regulation sequence which targets the expression of the DNA encoding the recombinant polypeptide to a particular cell or tissue type, e.g. expression of human serum albumin in the liver of a transgenic bovine species. When the recombinant polypeptide is to be secreted from such targeted cells or tissues, a secretory DNA sequence encoding a secretory signal sequence functional in the particular targeted cell or tissue is operably linked to the recombinant DNA sequence encoding the recombinant polypeptide, e.g. secretion of human serum albumin from bovine liver into the bovine circulatory system.

Further, the invention includes methods for producing transgenic bovine species having a desirable phenotype. The method can comprise first causing the methylation of a transgene capable of conferring the desirable phenotype when incorporated into the cells of a transgenic bovine, e.g., by transforming an appropriate bacterium, such as E. coli MM 294, with a plasmid containing the transgene. The methylated transgene is then excised and introduced into fertilized oocytes of the animal to permit integration into the genome. The oocytes are then cultured to form pre-implantation embryos thereby replicating the genome of each of the fertilized oocytes. Thereafter, at least one cell is removed from each of the pre-implantation embryos and treated to release the DNA contained therein. Each of the released DNAs are then digested with a restriction endonuclease capable of cleaving the methylated form of the transgene but incapable of cleaving the unmethylated form of the transgene formed after integration into and replication of the genomic DNA. Those pre-implantation embryos which have integrated the transgene contain DNA which is resistant to cleavage by the restriction endonuclease in the region containing the transgene. This resistance to digestion, which can be detected by electrophoresis of the digest after PCR amplification of the DNA and hybridization with a labelled probe for the transgene, facilitates the identification of successful transgenesis.

The invention also includes a method to produce a population of transgenic bovine offspring having the same genotype. This method utilizes a specific embodiment of the above method for detecting early transgenesis. In this method, a methylated transgene is introduced into fertilized oocytes which are cultured to pre-implantation embryos. Thereafter, each pre-implantation embryo is divided to form first and second hemi-embryos. Each of the first hemi-embryos are then analyzed for transgenesis as described above. After identifying successful transgenesis in at least one first hemi-embryo, the second untreated hemi-embryo which contains the integrated transgene, is cloned to form a multiplicity of clonal transgenic blastocysts or hemi-blastocysts, each of which have the same genotype. The transgenic embryos are thereafter transplanted into one or more recipient female parents to produce a population of transgenic bovines having the same genotype.

### Brief Descripton of the Drawings

The accompanying drawings, which are incorporated in and form a part of the specification, illustrate embodiments of the present invention and, together with the description, serve to explain the principles of the invention. In the drawings:

Fig. 1 depicts the DNA (Seq. ID No.: 1) and amino acid (Seq. ID No.: 2) sequence for a human lactoferrin clone derived from a human mammary cDNA library as described herein except that the sequence between nucleotides 1557-1791 and 2050-2119 corresponds to the previously published sequence (Rado et al., (1987) Blood 70, 989-993).

Fig. 2 depicts the complete DNA (Seq. ID No.: 3) and amino acid (Seq. ID No.: 4) sequence of human lactoferrin including 5' and 3' untranslated sequence as well as the complete human lactoferrin signal sequence.

Fig. 3 is a restriction map of a clone of a 5'-flanking region of bovine αS1-casein gene.

Fig. 4 is a restriction map of a clone of a 3'-flanking region of bovine αS1-casein gene.

Figs. 5A, 5B and 5C depict the construction of pSI3'5'CAT and pSI5'CAT.

Fig. 6 depicts pMH-1.

Figs. 7A through 7F depict the construction of expression vectors containing sequences encoding human lactoferrin.

Fig. 8 depicts an alternate pathway for the construction of a transgene of the invention encoding human lactoferin.

Fig. 9 depicts the construction of a plasmid pPC containing a transgene encoding Protein C.

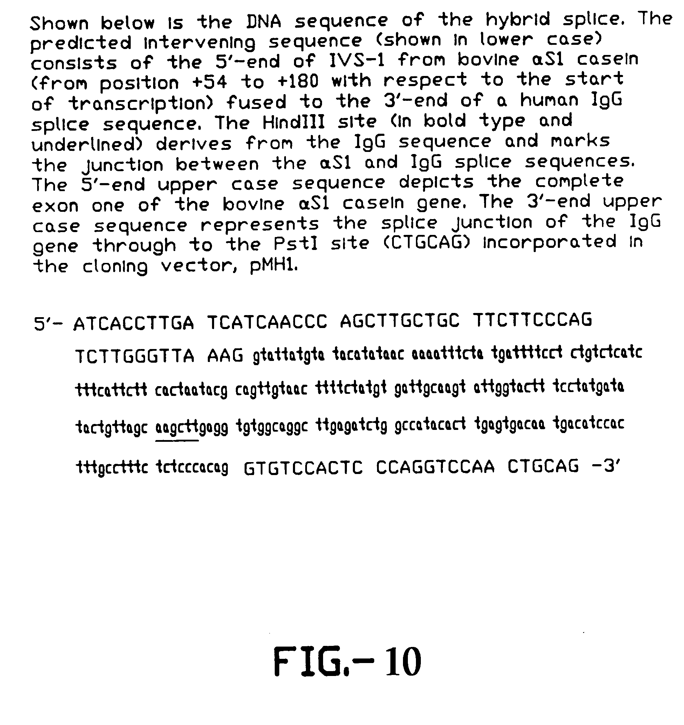

Fig. 10 depicts the DNA sequence for a hybrid intervening sequence used in a preferred embodiment of the invention. This hybrid sequence comprises a 5' portion from an intervening sequence of bovine αS1-casein and a 3' portion from an intervening sequence of an IgG intervening sequence. The juncture of the 5' and 3' portion is the HindIII site shown.

### Detailed Description of the Invention

Desirable phenotypes for transgenic bovine species include, but are not limited to, the production of recombinant polypeptides in the milk of female transgenic animals, the production of animal models for the study of disease, the production of animals with higher resistance to disease (e.g. diseases of the mammary gland such as mastitis) and the production of recombinant polypeptides in the blood, urine or other suitable body fluid or tissue of the animal. In the preferred embodiments, transgenic bovine species are made which are capable of producing recombinant human lactoferrin, human serum albumin and human Protein C in the milk of lactating females or human serum albumin in the liver of the transgenic animal.

The transgenic bovine species produced by the practice of the invention are produced by introducing a "transgene" into an embryonal target cell of the animal. A transgene is a DNA sequence which is capable of producing a desirable phenotype when contained in the genome of cells of a transgenic bovine species. In specific embodiments, the transgene comprises a "recombinant DNA sequence" encoding a "recombinant polypeptide". In such cases, the transgene is capable of being expressed to produce the recombinant polypeptide.

As used herein, a "recombinant polypeptide" (or the recombinant DNA sequence encoding the same) is either a "heterologous polypeptide" or a "homologous polypeptide". Heterologous polypeptides are polypeptides which are not normally produced by the transgenic animal. Examples of heterologous polypeptides include human milk proteins such as lactoferrin, lysozyme, secreted immunoglobulins, lactalbumin, bile salt-stimulated lipase, etc., human serum proteins such as albumin, immunoglobulins, Factor VIII, Factor IX, protein C, etc. and industrial enzymes such as proteases, lipases, chitinases, and liginases from procaryotic and eucaryotic sources. The recombinant DNA sequences include genomic and cDNA sequences encoding the recombinant polypeptide.

When recombinant DNA sequences encoding a heterologous polypeptide are used, the transgene may be integrated in a random manner into the genome of the species used for transgenesis. As disclosed in the Examples, transgenes encoding human lactoferrin, human serum albumin and human Protein C in conjunction with a αS1-casein secretory signal sequence under control of αS1-casein expression regulation sequences are designed to produce and secrete these heterologous polypeptides from the mammary gland of a lactating transgenic bovine into its milk.

As used herein, a homologous polypeptide is one which is endogenous to bovine species. Examples of endogenous polypeptides from bovine species include bovine milk proteins such as αS1, αS2, β- and κ-casein, β-lactoglobulin lactoferrin, lysozyme, cholesterol hydrolase, serum proteins such as serum albumin and proteinaceous hormones such as growth hormones. When recombinant DNA sequences encoding a homologous polypeptide are used, the transgene is preferably integrated in a random manner into the genome of the species used for transgenesis. Such random integration results in a transgenic animal which contains not only the transgene encoding the endogenous polypeptide but also the corresponding endogenous genomic DNA sequence. Accordingly, such transgenic bovine species are readily characterized by an increase in the copy number of genes encoding the endogenous polypeptide. Further, the transgene will generally be located at a position which is different from the endogenous gene.

When DNA encoding a homologous polypeptide is expressed in bovine species, the transgenic animal is characterized by an increase in the amount of the homologous polypeptide in either the endogenous tissue or fluid in which it is normally found and/or by its presence in a tissue and/or body fluid which either does not normally contain the homologous polypeptide or produces it at significantly lower levels.

Thus, for example, bovine cholesterol hydrolase is normally present in the colostrum for about the first 15-20 days of lactation. This naturally occurring endogenous polypeptide increases calf weight. This protein, however, is also a homologous polypeptide when, for example, its expression in mammary secretory cells is placed under the control of expression regulation sequences, such as those obtained from bovine casein genes, which facilitate the expression of the homologous polypeptide beyond the lactation period that it is normally present. Thus, according to one aspect of the invention, bovine cholesterol hydrolase expression is maintained in transgenic bovine milk by placing the expression of cholesterol hydrolase recombinant DNA (either cDNA or genomic) under the control of bovine αS1-casein expression regulation sequences. When a genomic recombinant DNA is used, it is engineered such that it has appropriate restriction sites (e.g. ClaI and SalI) at the 5' and 3' end of the structural gene such that it is capable of being inserted into an appropriate transgene genomic cassette (e.g. p-16 kb, CS which is described in Example 15). Alternatively, a recombinant DNA encoding bovine cholesterol hydrolase derived from cDNA may be placed under control of bovine αS1-casein expression regulation sequence by substituting the human lactoferrin sequences in a plasmid such as p16, 8HLF3 (containing a hybrid intervening sequence) or p16, 8HLF4 (containing a homologous αS1-casein intervening sequence). When these particular plasmids are used, the cDNA clone is engineered such that it has appropriate ClaI and SalI restriction sites at the ends of the recombinant DNA.

By way of further example, bovine lactoferrin is normally present in only trace amounts in cow's milk. When, however, bovine lactoferrin is expressed under control of other regulatory sequences, for example, obtained from an αS1-casein gene, higher amounts of lactoferrin in the milk of transgenic bovine species are obtained. In another example, a transgene comprising DNA encoding homologous bovine growth hormone is incorporated into the bovine genome to confer superior growth characteristics to the transgenic animal. In other instances, homologous polypeptides include, for example, a polypeptide which normally is maintained intracellularly in a particular species but which is secreted into the milk or other extracellular compartment of the transgenic species, such as the circulatory system.

Each of the heterologous or homologous polypeptides are characterized by specific amino acid and nucleic acid sequences. It is to be understood, however, that such sequences include naturally occurring allelic variations thereof and variants produced by recombinant methods wherein such nucleic acid and polypeptide sequences have been modified by the substitution, insertion and/or deletion of one or more nucleotides in such nucleic acids to cause the substitution, insertion or deletion of one ore more amino acid residues in the recombinant polypeptide.

When expression of the DNA of the transgene is necessary to generate a desired phenotype, e.g. to produce a recombinant polypeptide, the transgene typically includes at least a 5' and preferably additional 3' "expression regulation sequences" each operably linked to a recombinant or secretory-recombinant DNA as defined hereinafter. Such expression regulation sequences in addition to controlling transcription also contribute to RNA stability and processing, at least to the extent they are also transcribed.

Such expression regulation sequences are chosen to produce tissue-specific or cell type-specific expression of the recombinant or secretory-recombinant DNA. Once a tissue or cell type is chosen for expression, 5' and optional 3' expression regulation sequences are chosen. Generally, such expression regulation sequences are derived from genes that are expressed primarily in the tissue or cell type chosen. Preferably, the genes from which these expression regulation sequences are obtained are expressed substantially only in the tissue or cell type chosen, although secondary expression in other tissue and/or cell types is acceptable if expression of the recombinant DNA in the transgene in such tissue or cell type is not detrimental to the transgenic animal. Particularly preferred expression regulation sequences are those endogenous to bovine species.

However, expression regulation sequences from other species such as those from human genes may also be used. In some instances, the expression regulation sequences and the recombinant DNA sequences (either genomic or CDNA) are from the same species, e.g., each from bovine species or from a human source. In such cases, the expression regulation sequence and the recombinant DNA sequence are homologous to each other. Alteratively, the expression regulation sequences and recombinant DNA sequences (either cDNA or genomic) are obtained from different species, e.g., an expression regulation sequence from bovine species and a recombinant DNA sequence from a human source). In such cases, the expression regulation and recombinant DNA sequence are heterologous to each other. The following defines expression regulation sequences from endogenous genes. Such definitions are also applicable to expression regulation sequences from non-endogenous, heterologous genes.

In general, the 5' expression regulation sequence includes the transcribed portion of the endogenous gene upstream from the translation initiation sequence (the 5' untranslated region or 5' UTR) and those flanking sequences upstream therefrom which comprise a functional promoter. As used herein, a "functional promoter" includes those necessary untranscribed DNA sequences which direct the binding of RNA polymerase to the endogenous gene to promote transcription. Such sequences typically comprise a TATA sequence or box located generally about 25 to 30 nucleotides from the transcription initiation site. The TATA box is also sometimes referred to the proximal signal. In many instances, the promoter further comprises one or more distal signals located upstream from the proximal signal (TATA box) which are necessary to initiate transcription. Such promoter sequences are generally contained within the first 100 to 200 nucleotides located upstream from the transcription initiation site, but may extend up to 500 to 600 nucleotides from the transcription initiation site. Such sequences are either readily apparent to those skilled in the art or readily identifiable by standard methods. Such promoter sequences alone or in combination with the 5' untranslated region are referred to herein as "proximal 5' expression regulation sequences".

In addition to such proximal 5' expression regulation sequences, it is preferred that additional 5' flanking sequences (referred to herein as "distal 5' expression regulation sequences") also be included in the transgene. Such distal 5' expression regulation sequences are believed to contain one or more enhancer and/or other sequences which facilitate expression of the endogenous gene and as a consequence facilitate the expression of the recombinant or secretory-recombinant DNA sequence operably linked to the distal and proximal 5' expression regulation sequences. The amount of distal 5' expression regulation sequence depends upon the endogenous gene from which the expression regulation sequences are derived. In general, however, such sequences comprise 5' flanking regions of approximately lkb, more preferably 16kb and most preferably about 30kb of 5' flanking sequence. The determination of the optimal amount of distal 5' expression regulation sequence used from any particular endogenous gene is readily determined by varying the amount of distal 5' expression regulation sequence to obtain maximal expression. In general, the distal 5' expression regulation sequence will not be so large as to extend into an adjacent gene and will not include DNA sequences which adversely effect the level of transgene expression.

In addition, it is preferred that 3' expression regulation sequences also be included to supplement tissue or cell-type specific expression. Such 3' expression regulation sequences include 3' proximal and 3' distal expression regulation sequences from an appropriate endogenous gene. The 3' proximal expression regulation sequences include transcribed but untranslated DNA positioned downstream from the translation stop signal in the recombinant DNA sequence (also referred to as the 3' untranslated region or 3' UTR). Such sequences generally terminate at a polyadenylation sequence (either from the endogenous gene or from other sources such as SV40) and sequences that may affect RNA stability. Generally, 3' UTR's comprise about 100 to 500 nucleotides downstream from the translation stop signal in the gene from which the 3' regulation sequence is derived. Distal 3' expression regulation sequences include flanking DNA sequences downstream from the proximal 3' expression regulation sequence. Some of these distal sequences are transcribed, but do not form part of the mRNA while other sequences in this distal 3' expression regulation sequence are not transcribed at all. Such distal 3' expression regulation sequences are believed to contain enhancer and/or other sequences which enhance expression. Such sequences are believed to be necessary for efficient polydenylation and contain transcription termination sequences Preferably, such sequences comprise about 2kb, more preferably 8kb and most preferably about 15kb of 3' flanking sequence.

Although the use of both 5' and 3' expression regulation sequences are preferred, in some embodiments of the invention, endogenous 3' regulation sequences are not used. In such cases, the 3' proximal expression regulation sequences normally associated with the genomic DNA encoded by the recombinant DNA sequence are used to direct polyadenylation. In addition, distal 3' regulation sequences from the genomic DNA encoding the recombinant polypeptide may also be employed preferably in the same amounts as set forth for endogenous 3' expression regulation sequences. In such cases, it is to be understood that the recombinant polypeptide encoded by the transgene may comprise either genomic DNA or a double stranded DNA derived from cDNA. As with the 5' expression regulation sequences, the optimal amount of 3' expression regulation sequence may be readily determined by varying the amount of 3' flanking sequence to obtain maximal expression of the recombinant polypeptide. In general, the distal 3' regulation sequence, be it from an endogenous gene or a heterologous gene, will not extend into the adjacent gene from which is derived and will exclude any sequences which adversely effect the level of transgene expression.

Examples of expression regulation sequences are provided in Table I.

**TABLE 1**

| Expression Regulation Sequence | Tissue Specificity | Animal Species |
|---|---|---|
| 16kb of bovine αS1 casein 5' to structural gene and 8kb 3' to structural gene | Mammary secretory cells | bovine |
| ≈15kb 5' to albumin gene | Liver | murine |
| ≈15kb 5' to α-actin gene | Muscle | murine |
| ≈15kb upstream of protamine gene | Spermatids | murine |

In addition to the 5' and 3' expression regulation sequences and the recombinant DNA (either genomic or derived from cDNA) the transgenes of the invention preferably also comprise a "recombinant intervening sequence" which interrupts the transcribed but untranslated 5' region of the transgene. Such intervening sequences can be derived, for example, from bovine αS1-casein and from human lactoferrin. Such sequences as used herein are "homologous recombinant intervening sequences" in that the 5' and 3' RNA splice signals in such recombinant intervening sequences are those normally found in an intervening sequence from an endogenous or heterologous gene. Recombinant intervening sequences may, however, also comprise a "hybrid intervening sequence". Such hybrid intervening sequences comprise a 5' RNA splice signal and 3' RNA splice signal from intervening sequences from different sources. In some aspects of the invention, such hybrid intervening sequences comprise at least one "permissive RNA splice sequence". As used herein, a permissive RNA splice signal is an RNA splice signal sequence, preferably a 3' RNA splice signal, from an intron contained within a repertoire of germ line DNA segments which undergo rearrangement during cell differentiation. Examples of such gene repertoires include the immunoglobulin super gene family, including the immunoglobulins and T-cell antigen receptors as well as the repertoire of the major histocompatibility complex (MHC) genes and others. Particularly preferred permissive splice sequences are those obtained from the immunoglobulin repertoire, preferably of the IgG class, and more preferably those 3' splice signal sequences associated with the J-C segment rearrangement of the Ig heavy and light chain, most preferably the heavy chain. A particularly preferred permissive splice sequence comprises that portion of the sequence as shown downstream of the HindIII site in Fig. 11. A particularly preferred hybrid intervening sequence comprises the entire sequence shown in Fig. 11 which includes a 5' portion of an intervening sequence from bovine αS1-casein and a 3' sequence portion of an IgG heavy chain intervening sequence.

Such hybrid intervening sequences containing permissive RNA splice signals are preferably used when the recombinant DNA corresponds to a cDNA sequence.

However, such hybrid intervening sequences are not limited to transgenes utiiizing cDNA sequence. Rather, hybrid intervening sequences are also useful when the recombinant polypeptide is encoded by a genomic sequence. Based on the results obtained with the cDNA recombinant DNA and the general expectation that genomic DNA sequences express at higher levels than sequences derived from cDNA, it is expected that such hybrid intervening sequences used in conjunction with genomic recombinant DNA will further enhance expression levels above that which would otherwise be obtained with genomic sequence alone.

Based on the foregoing, it is apparent that preferred transgenes include large amounts of 5' and 3' expression regulation sequences. Further, the recombinant DNA is preferably derived from genomic clones which may be tens to hundreds of kilobases in length. Based on the present technology for cloning and manipulating DNA, the construction and microinjection of transgenes is practically limited to linearized DNA having a length not greater than about 50kb. However, the transgenes of the invention, especially those having a length greater than about 50kb, may be readily generated by introducing two or more overlapping fragments of the desired transgene into an embryonal target cell. When so introduced, the overlapping fragments undergo homologous recombination which results in integration of the fully reconstituted transgene in the genome of the target cell. In general, it is preferred that such overlapping transgene fragments have 100% homology in those regions which overlap. However, lower sequence homology may be tolerated provided efficient homologous recombination occurs. If non-homology does exist between the homologous sequence portions, it is preferred that the non-homology not be spread throughout the homologous sequence portion but rather be located in discrete areas. Although as few as 14 base pairs at 100% homology are sufficient for homologous recombination in mammalian cells (Rubnitz, J. and Subramani, S. (1984) Mol. Cell. Biol. 4, 2253-2258), longer homologous sequence portions are preferred, e.g. 500bp, more preferably 1000bp, next most preferably 2000bp and most preferably greater than 2000bp for each homologous sequence portion.

There is no known practical limit to the size of the transgene which may be formed using larger and/or greater numbers of overlapping transgene fragments. In particular, it is expected that transgenes may be formed by this approach having lengths between about 50 to 1000kb and more preferably between 50 and 500kb. Further, the use of homologous recombination of overlapping fragments is expected to be fruitful in the generation of transgenic bovine species, containing transgenes incorporating recombinant DNA comprising genomic DNA which otherwise could not be incorporated into a pronucleus to form a transgenic animal. Such genomic transgenes are expected to produce higher expression levels in transgenic cows as compared to that which is produced by transgenes encoding recombinant cDNA.

When, the ultimate object is to secrete a recombinant polypeptide, a "secretory DNA sequence" encoding a functional secretion signal peptide is also operably linked within the transgene to direct secretion of the recombinant polypeptide from one or more cell types within the transgenic animal. Secretory DNA sequences in general are derived from genes encoding secreted bovine proteins.

Such secretory DNA sequences are preferably derived from genes encoding polypeptides secreted from the cell type targeted for tissue-specific expression, e.g. secreted milk proteins for expression in and secretion from mammary secretory cells. Secretory DNA sequences, however, are not limited to such sequences. Secretory DNA sequences from proteins secreted from other cell types within the bovine species may also be used, e.g., the native signal sequence of a homologous gene encoding a protein secreted other than in the mammary glands. In addition, "heterologous secretory DNA sequences" which encode signal secretion peptides from species other than bovine may also be used e.g., human t-PA, human serum albumin human lactoferrin and human lactalbumin and secretion signals from microbial genes encoding secreted polypeptides such as from yeast, filamentous fungi, and bacteria. In general, a secretory DNA sequence may be defined functionally as any DNA sequence which when operably linked to a recombinant DNA sequence encodes a signal peptide which is capable of causing the secretion of the recombinant polypeptide.

In one of the preferred embodiments, a secretory DNA sequence encoding a secretory signal sequence functional in the mammary secretory cells of bovine species is used to cause secretion of recombinant polypeptide from bovine mammary secretory cells. The secretory DNA sequence is operably linked to the recombinant DNA sequence. Examples of such secretory DNA sequences include DNA sequences encoding signal secretion sequences for bovine αS1-casein, murine lactoferrin and human transferrin. The preferred secretory DNA sequence is that encoding the secretory sequence of αS1-casein from bovine species. The use of this secretory DNA sequence is described in more detail in the Examples.

"Operably linked" in the context of linking a secretory DNA sequence to a recombinant DNA sequence means that the secretory DNA sequence (comprising codons encoding the secretory signal peptide sequence) is covalently coupled to the recombinant DNA sequence so that the resultant secretory-recombinant DNA sequence encodes 5' to 3' for the secretory signal sequence and recombinant polypeptide. Accordingly, the reading frame for the secretory sequence and the recombinant DNA sequence must be covalently combined such that an open reading frame exists from the 5' end of the mRNA sequence formed after transcription and processing of the primary RNA transcript. This open reading frame in the RNA contains a 5' sequence portion encoding the secretory signal peptide and a 3' sequence portion encoding the recombinant polypeptide. When so constructed, the recombinant polypeptide produced upon expression of the secretory-recombinant DNA sequence is of a form which is capable of being secreted from targeted cells which express the DNA sequence. The signal peptide generally is removed in vivo during secretion to produce an extracellular form of the recombinant polypeptide.

In the preferred embodiments of the invention, a secretory-recombinant DNA sequence is expressed predominantly in the mammary secretory cells of transgenic bovine species. Such tissue-specific expression is obtained by operably linking mammary specific expression regulation DNA sequences to the above secretory-recombinant DNA sequence. Such mammary specific regulation sequences include the aforementioned regulation sequences contained in various bovine genes preferentially expressed in the mammary secretory cells of the species. Such mammary specific genes include αS1-casein; αS2-casein; β-casein; K-casein; α-lactalbumin; and β-lactoglobulin. Preferred expression regulation sequences are derived from αS1-casein as described more in detail in the Examples.

In general, the transgenes of the invention that are designed to secrete the recombinant polypeptide into transgenic bovine milk are capable of causing such secretion at levels significantly higher than that previously reported for transgenic mice and sheep. When the recombinant polypeptide is encoded by a recombinant DNA corresponding to, or derived from, cDNA, the molar concentration of the recombinant polypeptide is preferably greater than about 1.0 µM, more preferably greater than about 100 µM, and most preferably greater than 100 µM. When viewed from the perspective of the level of recombinant polypeptide present in the transgenic milk, the amount of recombinant polypeptide is preferably greater than 50 µg/ml, more preferably greater than about 500 µg/ml and most preferably greater than about 1000 µg/ml (1mg/ml).

When the transgene of the invention encodes a recombinant polypeptide that is encoded by recombinant DNA derived from or corresponding to genomic DNA (or comprised substantially of such genomic sequences, e.g. greater than about 50%, more preferably greater than about 75%, most preferably greater than 90% of the codons encoding the recombinant polypeptide are from genomic sequences), the molar concentrations and protein levels in bovine transgenic milk are the same as for cDNA or higher. In general, the molar concentration of the recombinant polypeptide in such transgenic milk is preferably greater than about 50 µM, more preferably greater than about 150 µM, most preferably greater than about 500 µM. When viewed from the level of protein in the transgenic milk, the levels are preferably greater than about 10 mg/ml, more preferably greater than about 2.5 mg/ml, most preferably greater than 5 mg/ml.

The foregoing molar concentration and protein levels in bovine transgenic milk will vary depending upon the molecular weight of the particular recombinant polypeptide. A particular advantage of producing a recombinant polypeptide in bovine transgenic milk is that relatively large molecular weight polypeptides may be so produced which are otherwise difficult to produce in large quantities in other systems such as prokaryotic expression systems. Although any recombinant polypeptide may be produced in bovine transgenic milk according to the invention, it is generally preferred that such recombinant polypeptides have a molecular weight greater than about 10,000 Daltons. However, other recombinant polypeptides having molecular weights of greater than 15,000, greater than 20,000 and greater than 60,000 Daltons may also be expressed in transgenic bovine milk. For example, human lysozyme having a molecular weight of 17,000 Daltons and lactoferrin having a molecular weight of 79,000 Daltons may be readily produced in the transgenic milk of bovine species according to the disclosure of the invention. Thus, the recombinant polypeptides of the invention have a wide range of molecular weights.

As a consequence, the foregoing preferred molar concentrations of recombinant polypeptides are adjusted when higher molecular weight recombinant polypeptides are produced. Such adjustment is made by converting the molar concentration to the amount of protein produced and adjusting the molar concentrations so that the recombinant protein level is within the following preferred concentrations.

Most of the previous reports relating to the production of polypeptides in transgenic milk involve transgenic mice. The mouse, however, normally produces between 55 to 80 milligrams of protein per ml of milk. A cow, on the other hand, normally produces between 30 to 34 milligrams of protein per ml. Since exceptionally high levels of recombinant polypeptide production may adversely affect the production of endogenous milk protein and/or have adverse effects upon the mammary secretory gland, it is preferred that the recombinant polypeptide concentration be between about 3 and 50% of the normal bovine milk protein concentration (i.e., between about 1 and 17 milligrams of recombinant polypeptide per ml of transgenic milk), more preferably between 10 to 20% (i.e., between 3 to about 7 milligrams per ml) and most preferably between 10 and 15% (i.e., between about 3 and 5 milligrams per ml) of the normal amount of protein produced in bovine milk. Such preferred ranges also provide a preferred maximum limit to the aforementioned levels of protein produced in transgenic bovine milk.

The above described linking of various DNA sequences to form the transgene of the invention are performed by standard methods known to those skilled in the art or as described herein. Once the transgene or overlapping homologous fragments encoding the transgene are constructed as described they are used to make transgenic non-human animals.

Methods of introducing transgenes or overlapping transgene fragments into embryonal target cells include microinjection of the transgene into the pronuclei of fertilized oocytes or nuclei of ES cells of a bovine species. Such methods for murine species are well known to those skilled in the art. Alternatively, the transgene may be introduced into an animal by infection of zygotes with a retrovirus containing the transgene (Jaenisch, R. (1976), Proc. Natl. Acad. Sci. USA, 73, 1260-1264). The preferred method is microinjection of the fertilized oocyte. In this preferred embodiment, the fertilized oocytes are first microinjected by standard techniques. They are thereafter cultured in vitro until a "pre-implantation embryo" is obtained. Such pre-implantation embryos preferably contain approximately 16 to 150 cells. The 16 to 32 cell stage of an embryo is commonly referred to as a morula. Those pre-implantation embryos containing more than 32 cells are commonly referred to as blastocysts. They are generally characterized as demonstrating the development of a blastocoel cavity typically at the 64 cell stage. Methods for culturing fertilized oocytes to the pre-implantation stage include those described by Gordon, et al. (1984), Methods in Enzymology, 101, 414; Hogan, et al. (1986) in Manipulating the Mouse Embryo, Cold Spring Harbor Laboratory Press, Cold Spring Harbor, N.Y. (for the mouse embryo); and Hammer, et al. (1985), Nature, 315, 680 (for rabbit and porcine embryos) Gandolfi et al. (1987) J. Reprod. Fert. 81, 23-28; Rexroad et al. (1988) J. Anim. Sci. 66, 947-953 (for ovine embryos) and Eyestone, W.H. et al. (1989), J. Reprod. Fert., 85, 715-720; Camous., et al. (1984), J. Reprod. Fert., 72, 779-785; and Heyman, Y., et al. (1987), Theriogenology, 27, 5968 (for bovine embryos). Such pre-implantation embryos are thereafter transferred to an appropriate female by standard methods to permit the birth of a transgenic or chimeric animal depending upon the stage of development when the transgene is introduced. As is well known, mosaic animals can be bred to form true germline transgenic animals.

Since the frequency of transgene incorporation is often low, the detection of transgene integration in the pre-implantation embryo is highly desirable. In one aspect of the invention methods are provided for identifying embryos wherein transgenesis has occurred and which permit implantation of transgenic embryos to form transgenic animals. In this method, one or more cells are removed from the pre-implantation embryo. When equal division is used, the embryo is preferably not cultivated past the morula stage (32 cells). Division of the pre-implantation embryo (reviewed by Williams et al. (1986) Theriogenology 22, 521-531) results in two "hemi-embryos" (hemi-morula or hemi-blastocyst) one of which is capable of subsequent development after implantation into the appropriate female to develop in utero to term. Although equal division of the pre-implantation embryo is preferred, it is to be understood that such an embryo may be unequally divided either intentionally or unintentionally into two hemi-embryos which are not necessarily of equal cell number.

Essentially, all that is required is that one of the embryos which is not analyzed as hereinafter described be of sufficient cell number to develop to full term in utero. In a specific embodiment, the hemi-embryo which is not analyzed as described herein, if shown to be transgenic, is used to generate a clonal population of transgenic non-human animals.

One of each of the hemi-embryos formed by division of pre-implantation embryos is analyzed to determine if the transgene has been integrated into the genome of the organism. Each of the other hemi-embryos is maintained for subsequent implantation into a recipient female of the species. A preferred method for detecting transgenesis at this early stage in the embryo's development uses these hemi-embryos in connection with a unique property of the restriction endonuclease Dpn I. This enzyme recognizes the sequence GATC in doublestranded DNA but only when the adenine in each strand within this sequence is methylated at N-6. When using this preferred method, the transgene containing the sequence GATC is methylated prior to microinjection either by transferring the transgene on an appropriate plasmid through a DAM⁺ strain of microorganisms such as E. coli MM294 or by directly methylating the transgene with dam methylase. The methylated transgene (preferably without any exogenous sequences such as plasmid vector) is then microinjected into fertilized oocytes (approximately 10 to 500 copies per pronucleus, more preferably 50 to 100 copies per pronucleus). The fertilized oocytes so obtained are cultured in vitro to the pre-implantation stage. During this early growth and cell division phase, the genomic DNA is replicated. Accordingly, those copies of the methylated transgene integrated into the genome of the fertilized oocyte are unmethylated after replication whereas any non-integrated transgenes which may still exist after replication will remain methylated. (Lacks, S., et al. (1977), J. Mol. Biol., 114, 153.) This differential methylation pattern for integrated versus non-integrated transgene permits the identification of which fertilized oocytes have integrated the transgene into the genome.

The identification of the pre-implantation embryos containing the integrated transgene is achieved by analyzing the DNA from each of the hemi-embryos, Such DNA is typically obtained by lysing the hemi-embryo and analyzing the thus released DNA after treatment as described by Ninomiy, T. et al. (1989) molecular Reproduction and Development 1, 242-248. Each of the DNA samples is treated with Dpn I. Thereafter, a polymerase chain reaction (Saiki, et al. (1985), Science, 230, 1350-1354) is preformed to amplify all or part of the transgene. When the entire transgene is amplified, two extension primers each complimentary to opposite strands at opposing ends of the transgene are used for amplification. When, however, less than the entire transgene is amplified, such extension primers are chosen such that the amplified gene product spans the Dpn I site in the transgene. If Dpn I cleavage has not occurred, PCR amplification results in amplified sequences having a predetermined size whereas primer extension for those transgenes which have been cleaved will not result in exponential amplification. Generally, the Dpn I/PCR amplified DNA from the hemi-embryo is subjected to electrophoresis followed by hybridization with labeled probe complimentary to the region of the transgene between the two extension primers. This facilities the determination of the size of the amplified DNA sequences, if any, and provides an indication of whether the transgene has been integrated into the pre-implantation embryo from which the hemi-embryo was obtained (now called a "transgenic hemi-embryo"). If it has, the remaining untreated transgenic hemi-embryo is transplanted into a recipient parent. After in utero development, the transgenic non-human animal having the desired phenotype conferred by the integrated transgene is identified by an appropriate method in utero or after birth. Of course, other restriction endonucleases capable of cleaving a methylated DNA sequence but incapable of cleaving the unmethylated form of a recognition sequence may be used in the aforementioned method.

The above described method using Dpn I requires that the sequence GATC be present in the transgene of interest. In those cases when such a sequence is not present, it may be readily introduced into the transgene by site directed mutagenesis (Kunkei, T.A. (1985), Proc. Natl. Acad. Sci., 82, 488) or cassette mutagenesis (Wells, J.A., et al. (1985), Gene, 34, 315) provided such mutagenesis does not change the amino acid sequence encoded by the transgene (or causes an inconsequential change in amino acid sequence) and that any codons so generated are functional in the transgenic animal.

The above described methods for the detection of transgenesis in pre-implantation embryos provide economical and time saving method for generating transgenic non-human animals since they significantly decrease the number of pregnancies required to produce a transgenic animal and substantially increase the likelihood that an implanted embryo will produce a transgenic animal. Such methods are especially important for those bovine species for which very low or non-existent frequencies of transgenesis have been obtained.

In an alternate embodiment, the above described method for detecting transgenesis in pre-implantation embryos is combined with embryonic cloning steps to generate a clonal population of transgenic embryos which may thereafter be implanted into recipient females to produce a clonal population of transgenic animals also having the same genotype. In this regard, it is to be understood that transgenic embryos and/or non-human transgenic animals having the same "genotype" means that the genomic DNA is substantially identical between the individuals of the embryo and/or transgenic animal population. It is to be understood, however, that during mitosis various somatic mutations may occur which may produce variations in the genotype of one or more cells and/or animals. Thus, a population having the same genotype may demonstrate individual or subpopulation variations.

After a hemi-embryo is identified as a transgenic hemi-embryo, it is cloned. Such embryo cloning may be performed by several different approaches. In one cloning method, the transgenic hemi-embryo is cultured in the same or in a similar media as used to culture individual oocytes to the pre-implantation stage. The "transgenic embryo" so formed (preferably a transgenic morula) is then divided into "transgenic hemi-embryos" which can then be implanted into a recipient female to form a clonal population of two transgenic non-human animals. Alternatively, the two transgenic hemi-embryos obtained may be again cultivated to the pre-implantation stage, divided, and recultivated to the transgenic embryo stage. This procedure is repeated until the desired number of clonal transgenic embryos having the same genotype are obtained. Such transgenic embryos may then be implanted into recipient females to produce a clonal population of transgenic non-human animals.

In a preferred cloning method, the transgenic embryo is cloned by nuclear transfer according to the techniques of Prather et al. (1988) Biol. Reprod. 37, 59-86; Roble et al. (1987) J. Anim. Sci. 64, 642-664. According to this method, nuclei of the transgenic embryo are transplanted into enucleated oocytes, each of which is thereafter cultured to the blastocyst stage. At this point, the transgenic embryos may be resubjected to another round of cloning by nuclear transplantation or may be transferred to a recipient parent for production of transgenic offspring having the same genotype.

In addition to the foregoing methods for detecting early transgenesis, other methods may be used to detect transgenesis. Such methods include in utero and post partum analysis of tissue. In utero analysis is performed by several techniques. In one, transvaginal puncture of the amniotic cavity is performed under echoscopic guidance (Bowgso et al. (1975) Bet. Res. 96, 124-127; Rumsey et al. (1974) J. Anim. Sci. 39, 386-391). This involves recovering about 15 to 20 milliliters of amniotic fluid between about day 35 and day 100 of gestation. This volume of amniotic fluid contains about 1000 to 12,000 cells per ml originating from the urogenital tract, the skin and possibly the lungs of the developing embryo. Most of these cells are dead. Such cells, however, contain genomic DNA which is subjected to PCR analysis for the transgene as an indication of a successful transgenesis. Alternatively, fetal cells may be recovered by chorion puncture. This method also may be performed transvaginally and under echoscopic guidance. In this method, a needle is used to puncture the recipient animal's placenta, particularly the placentonal structures, which are fixed against the vaginal wall. Such sampling may be performed around day 60 of gestation in bovine species. Chorion cells, if necessary, are separated from maternal tissue and subjected to PCR analysis for the transgene as an indication of successful transgenesis.

Transgenesis may also be detected after birth. In such cases, transgene integration can be detected by taking an appropriate tissue biopsy such as from the ear or tail of the putative transgenic animal. About one to two centimeters of tail or about five to ten square millimeters of ear are obtained followed by southern blotting with a probe for the transgene according to the method of Hogan et al. (1986) Manipulating the Mouse Embryo, Cold Spring Harbor Laboratory.

In those embodiments where a recombinant polypeptide is expressed and secreted into the milk of transgenic bovine species, the transgenic milk so obtained may be either used as is or further treated to purify the recombinant polypeptide. This depends, in part, on the recombinant polypeptide contained in the transgenic milk and the ultimate use for that protein. Thus, when the recombinant polypeptide is secreted into transgenic milk to increase the nutritional value of the bovine milk, no further purification is generally necessary. An example of such a situation involves one of the preferred embodiments wherein human lactoferrin is produced in the milk of bovine species as a supplement to control intestinal tract infections in newborn human infants and to improve iron absorption. In other situations, a partial purification may be desired to isolate a particular recombinant polypeptide for its nutritional value. Thus, for example, human lactoferrin produced in transgenic bovine milk may be partially purified by acidifying the milk to about pH 4-5 to precipitate caseins. The soluble fraction (the whey) contains the human lactoferrin which is partially purified.

The recombinant polypeptide contained in bovine transgenic milk may also be used in food formulations. A particularly useful food formulation comprises an infant formula containing one or more recombinant polypeptides from transgenic bovine milk which have either nutritional or other beneficial value. For example, an infant formula containing human lactoferrin from transgenic bovine milk made according to the present invention provides a bacteriostatic effect which aids in controlling diarrhea in newborn. Similarly, recombinant polypeptides such as human casein and human lysozyme may also be generated in transgenic bovine milk to provide nutritional value. Table 2 sets forth the constituents of a typical infant formula. As indicated therein, the protein content varies between about 1.8 and 4.5 grams of protein per 100 kilocalories of formula. Thus, the total protein including recombinant polypeptide should lie between the values at least based on regulatory requirements in the United States from which the formulation in Table 2 is based. The amount of total protein including recombinant polypeptide, of course, may vary from the foregoing depending upon the local regulations where the particular formula is intended to be used.

**TABLE 2**

| Nutrient | Minimum^{a} | Maximum^{a} |
|---|---|---|
| Protein (gm)^{f} | 1.8^{b} | 4.5 |

| Fat: | | |
|---|---|---|
| gm | 3.3 | 6.0 |
| percent cal | 30.0 | 54.0 |

| Essential fatty acids (linoleate): | | |
|---|---|---|
| percent cal | 2.7 | |
| mg | 300.0 | |

| Vitamins: | | |
|---|---|---|
| (A) (IU) | 250.0 (75 µg)^{c} | 750.0 (225 µg)^{c} |
| D (IU) | 40.0 | 100.0 |
| K (µg) | 4.0 | |
| E (IU) | 0.7 (with 0.7 IU/gm lineoleic acid) | |
| | | |
| C (ascorbic acid (mg) | 8.0 | |
| B₁ (thiamine (µg) | 40.0 | |
| B₂ (riboflavin) (µg) | 60.0 | |
| B₄ (pyridoxine) (µg) | 35.0 (with 15 µg/gm of protein in formula) | |
| B₁₂ (µg) | 0.15 | |
| Niacin (µg) | 250.0 | |
| Folic acid (µg) | 4.0 | |
| Pantothenic acid (g) | 300.0 | |
| Biotin (µg) | 1.5^{d} | |
| Choline (mg) | 7.0^{d} | |
| Inositol (mg) | 4.0^{d} | |

| Minerals: | | |
|---|---|---|
| Calcium (mg) | 50.0^{e} | |
| Phosphorus (mg) | 25.0^{e} | |
| Magnesium (mg) | 6.0 | |
| Iron (mg) | 0.15 | |
| Iodine (µg) | 5.0 | |
| Zinc (mg) | 0.5 | |
| Copper (µg) | 60.0 | |
| Manganese (µg) | 5.0 | |
| Sodium (mg) | 20.0 | 60.0 |
| Potassium (mg) | 80.0 | 200.0 |
| Chloride (mg) | 55.0 | 150.0 |

| | | |
|---|---|---|
| ^{a}Stated per 100 kilocalories. | | |
| ^{b}The source of protein shall be at least nutritionally equivalent to casein. | | |
| ^{c}Retinol equivalents. | | |
| ^{d}Required to be included in this amount only in formulas which are not milk-based. | | |
| ^{e}Calcium to phosphorus ratio must be no less than 1.1 nor more than 2.0. | | |
| ^{f}Includes recombinant protein according to the invention or recombinant proteins and other proteins. | | |

In addition to infant formulas, other food formulations may also be supplemented with recombinant polypeptides from transgenic bovine milk. For example, such recombinant polypeptides may be used to supplement common diet formulations.

When the recombinant polypeptide is intended to be used pharmaceutically, purification methods consistent with such an application are called for. Such purification methods will depend on the particular recombinant polypeptide to be purified and are generally known to those skilled in the art. Such methods typically include a partial purification by casein fractionation followed by chromotography of the appropriate fraction containing the recombinant polypeptide. Such chromotography includes affinity chromotography, ion exchange chromotography, gel filtration and HPLC.

In a specific embodiment of the invention, transgenes are provided for producing human lactoferrin in the milk of transgenic bovine species. Human lactoferrin (HLF) is a single chain glycoprotein which binds two ferric ions. Secreted by exocrine glands (Mason et al. (1978) J. Clin. Path. 31, 316-327; Tenovuo et al. (1986) Infect. Immun. 51, 49-53) and polymorphonuclear neutrophil granulocytes (Mason et al. (1969) J. Exp. Med. 130, 643-658), this protein functions as part of a host non-specific defense system by inhibiting the growth of a diverse spectrum of bacteria. HLF exhibits a bacteriostatic effect by chelation of the available iron in the media, making this essential metal inaccessible to the invading microorganisms (Bullen et al. (1972) Br. Med. J. 1, 69-75; Griffiths et al. (1977) Infect. Immun. 15, 396-401; Spik et al. (1978) Immunology 8, 663-671; Stuart et al. (1984) Int. J. Biochem. 16, 1043-1947). This effect is blocked if the protein is saturated with ferric ions. Several studies suggest that HLF displays a direct bacteriocidal effect on certain microorganisms (Arnold et al. (1980) Infect. Immun. 28, 893-898; Arnold et al. (1977) Science 197, 263-265; Arnold et al. (1981) Infect. Immun. 32, 655-660; Arnold et al. (1982) Infect. Immun. 35, 792-797; Bortner et al. (1986) Infect. Immun. 51, 373-377). The bacteriocidal effect is also inhibited by iron saturation of the protein. No mechanism for the bactericidal effect of HLF has been postulated, although it has been demonstrated that it can damage the outer membrane and alter outer membrane permeability in gram-negative bacteria (Ellison et al. (1988) Infect. Immun. 56, 2774-2781).

Lactoferrin is the major iron binding protein in human milk (present at a concentration of about 1.5-1.7 mg/ml) and may play a role in the absorption of iron by the small intestine. All of the iron present in breast milk is thought to be bound to hLF and is taken up at very high efficiencies compared to formula (Hide, D.W., et al. (1981), Arch. Dis. Child., 56, 172). It has been postulated that the high uptake of the hLF bound iron is due to a receptor in the jejunum and data has been presented suggesting existence of receptors in Rhesus monkeys (Cox, et al. (1979), BBA, 588, 120; Davidson, L.A., et al. (1985), Fed. Proc., 18, 901). There is also evidence for specific lactoferrin receptors on mucosal cells of the small intestine of human adults (Cox, et al. (1979) Biochem. Biophvs. Acta. 588, 120-128). Free iron levels have been implicated in the control of the intestinal flora (Mevissen-Verhage, et al. (1985), Eur. J. Clin. Microbiol., 4, 14). Breast fed infants, compared with infants fed cow's milk, with and without added iron, were shown to have substantially reduced coliform and, elevated bifidobacteria and clostridia counts in fecal samples. In in vitro studies, human milk has been shown to have a specific inhibitory effect on E. coli (Brock, et al. (1983), Infect. and Immunit., 40, 453). Human milk has also been shown to have a specific inhibitory effect on E. coli in small intestine due to its high content of iron binding protein, predominantly hLF (Bullen, et al. (1972), British Med. J., i, 69).

Thus, the production of human lactoferrin in the milk of transgenic bovine species provides a source of human lactoferrin. Such lactoferrin may be purified from the transgenic milk for formulation purposes. Alternatively, the whole transgenic milk may be used, preferably after pasteurization, in either liquid or dried form. In addition, the beneficial action of human lactoferrin may be potentiated by combining the human lactoferrin or the transgenic milk containing it with human lysozyme. The human lysozyme may be simultaneously produced in the transgenic cow by introducing a second transgene simultaneously with the HLF transgene to produce a transgenic cow capable of producing more than one recombinant polypeptide in the transgenic milk. Alternatively, the transgenes may be sequentially introduced into bovine species. When such is the case, a transgenic bovine species is obtained containing one of the transgenes. Thereafter, embryonic cells, such as eggs, are obtained from the transgenic female and treated so as to incorporate the second transgene encoding the second polypeptide. Preferably, the egg is fertilized, followed by microinjection of the pronucleus of the zygote so obtained. It is to be understood that the foregoing combination of more than two recombinant polypeptides in transgenic bovine milk is not limited to the aforementioned human lactoferrin and lysozyme combination. Thus, the invention contemplates the production of transgenic bovine species and transgenic milk wherein more than one recombinant polypeptide is produced by such a transgenic animal in the transgenic milk.

The complete amino acid sequence of HLF has been determined (Metz-Boutigue et al. (1984) Eur. J. Biochem. 1451, 659-676). HLF comprises two domains, each containing one iron-binding site and one N-linked glycosylation site. These domains show homology between each other, indicative of an ancestral gene duplication and fusion event. In addition, HLF shares extensive homology with other members of the transferrin family (Metz-Boutigue, supra; Pentecost et al. (1987) J. Biol. Chem. 262, 10134-10139). Location of the amino acids involved in the iron-binding sites has been determined by X-ray crystallography (Anderson et al. (1987) Proc. Natl. Acad. Sci. 84, 1769-1773). A partial cDNA sequence for neutrophil HLF was published by Rado et al. (1987) Blood 70, 989-993. There was a >98% agreement between the amino acid sequence deduced from the cDNA and that which was determined by direct analysis of lactoferrin from human milk. The structure of the iron-saturated and iron-free form of human lactoferrin have recently been published. (Anderson, et al., (1989) J. Mol.Biol. 209, 711-734; Anderson, et al. (1990) Nature, 784-787.)

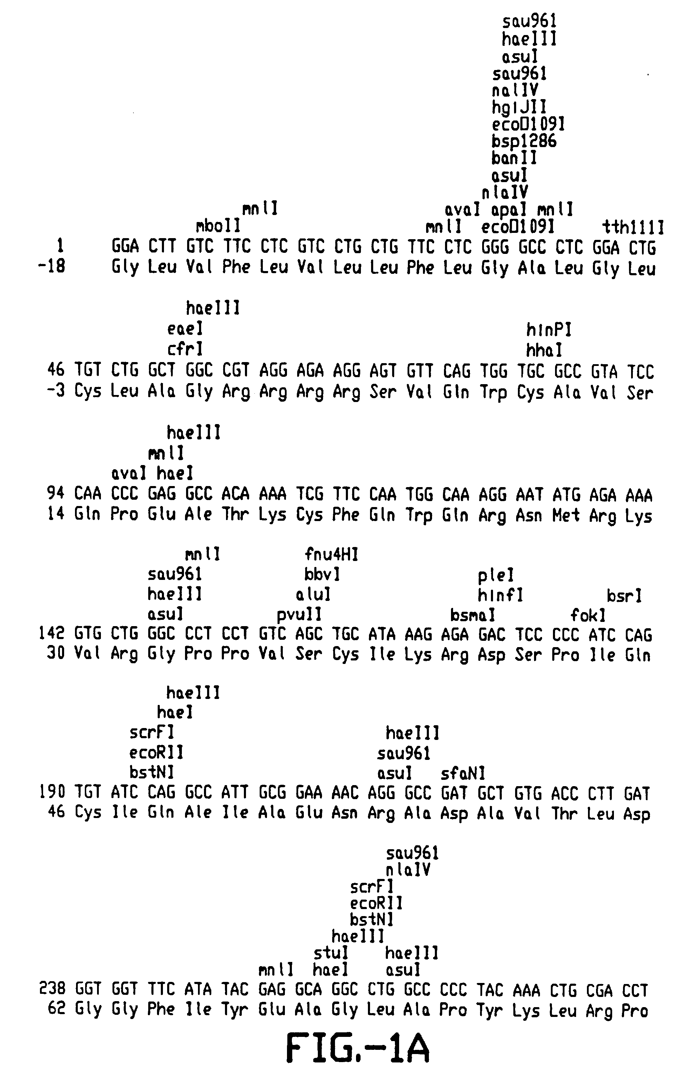

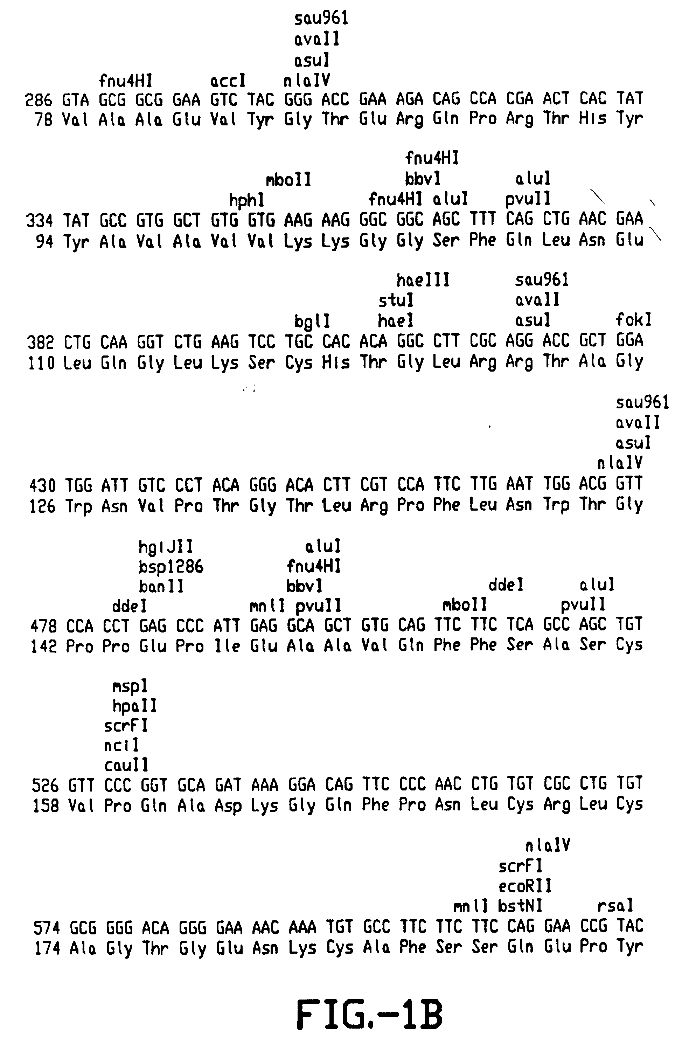

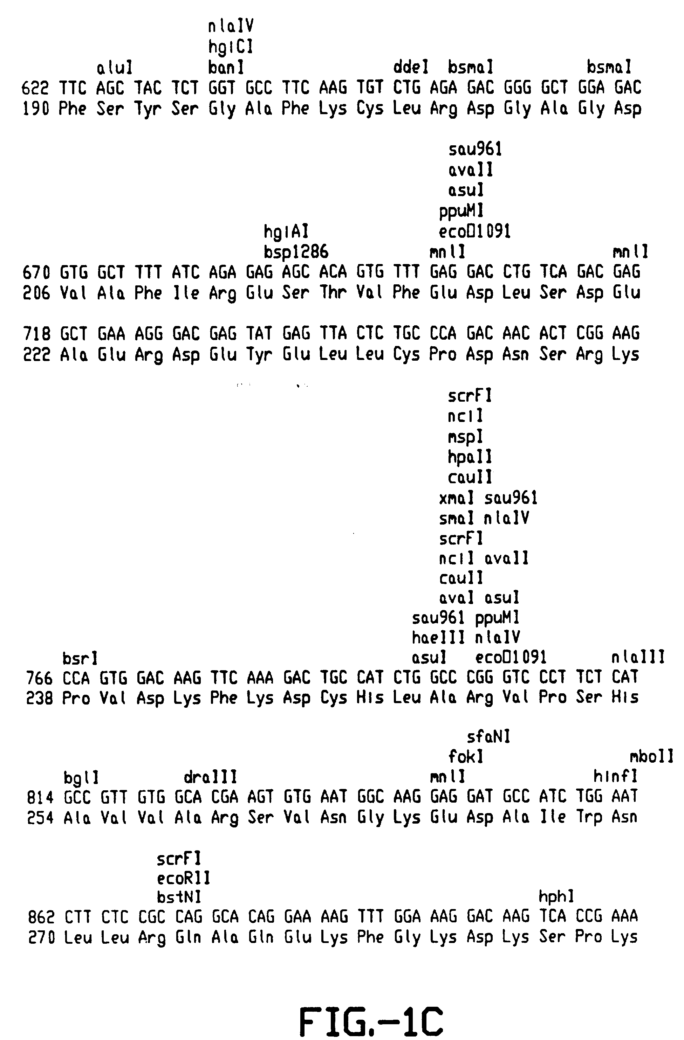

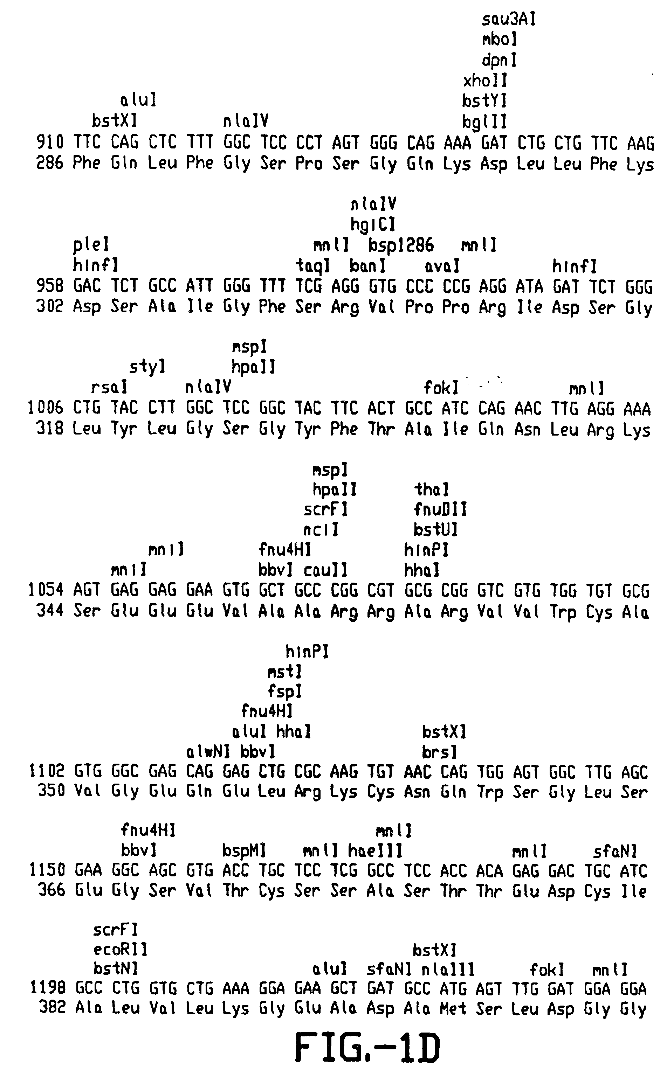

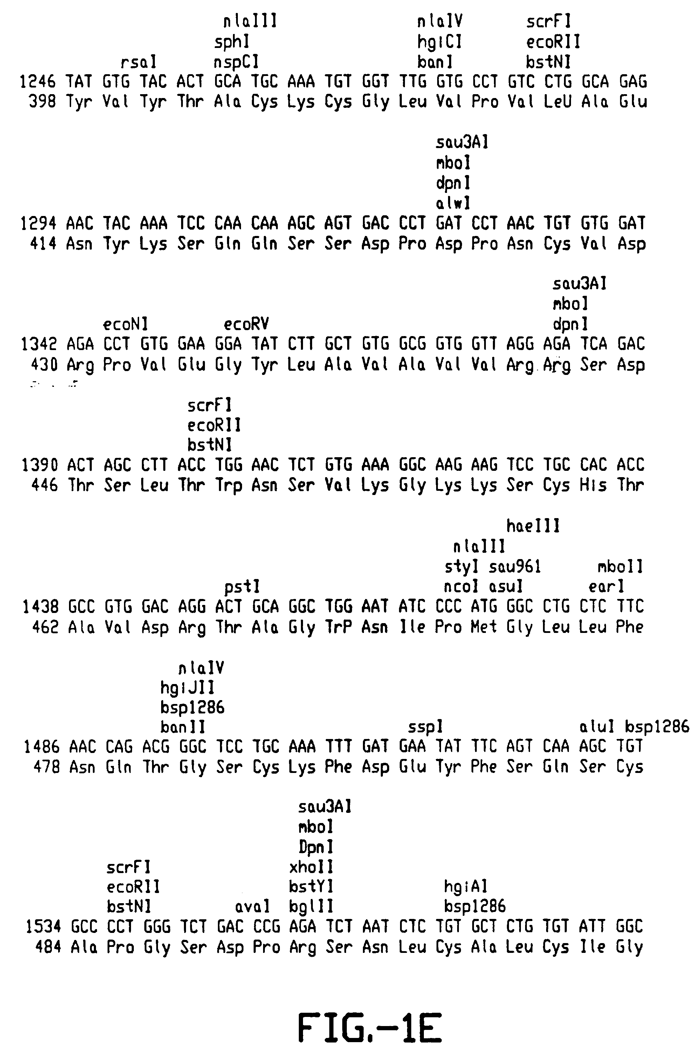

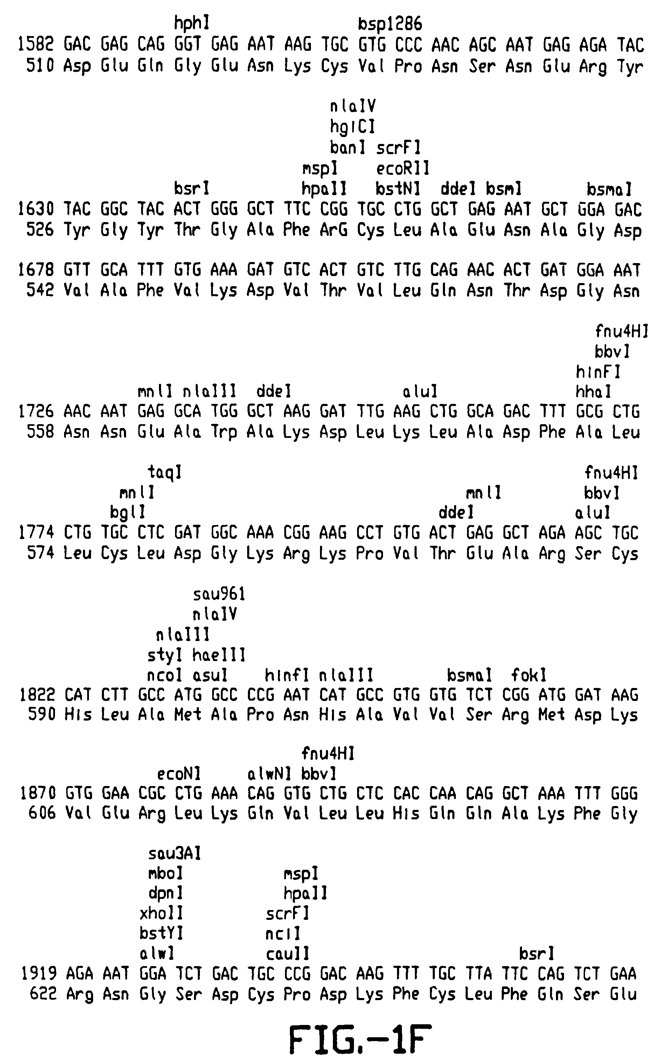

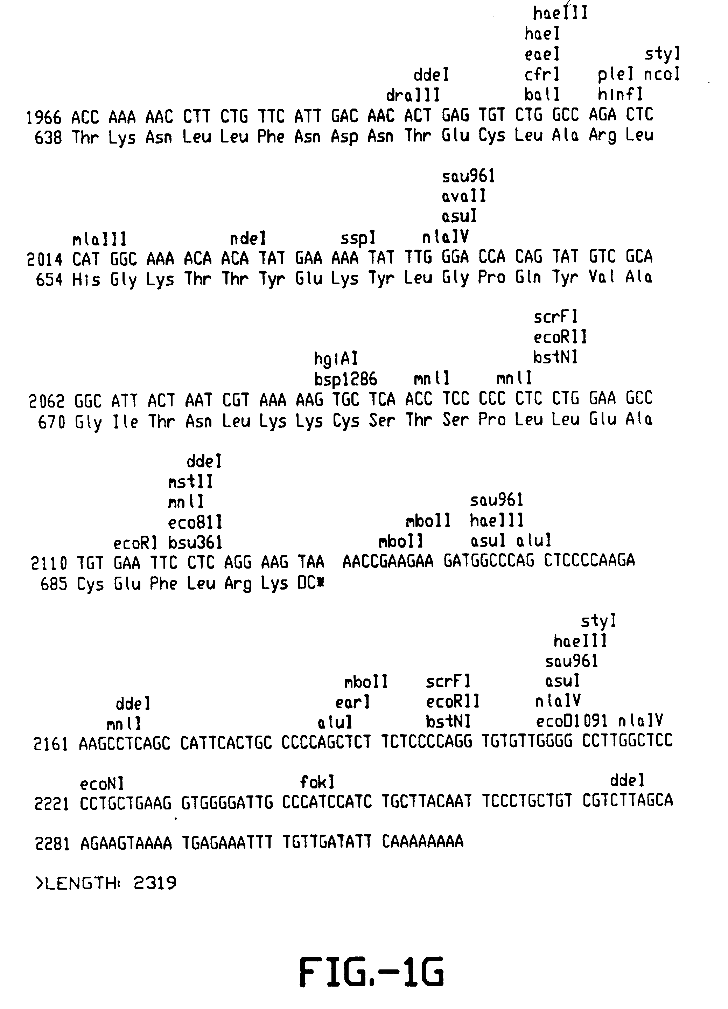

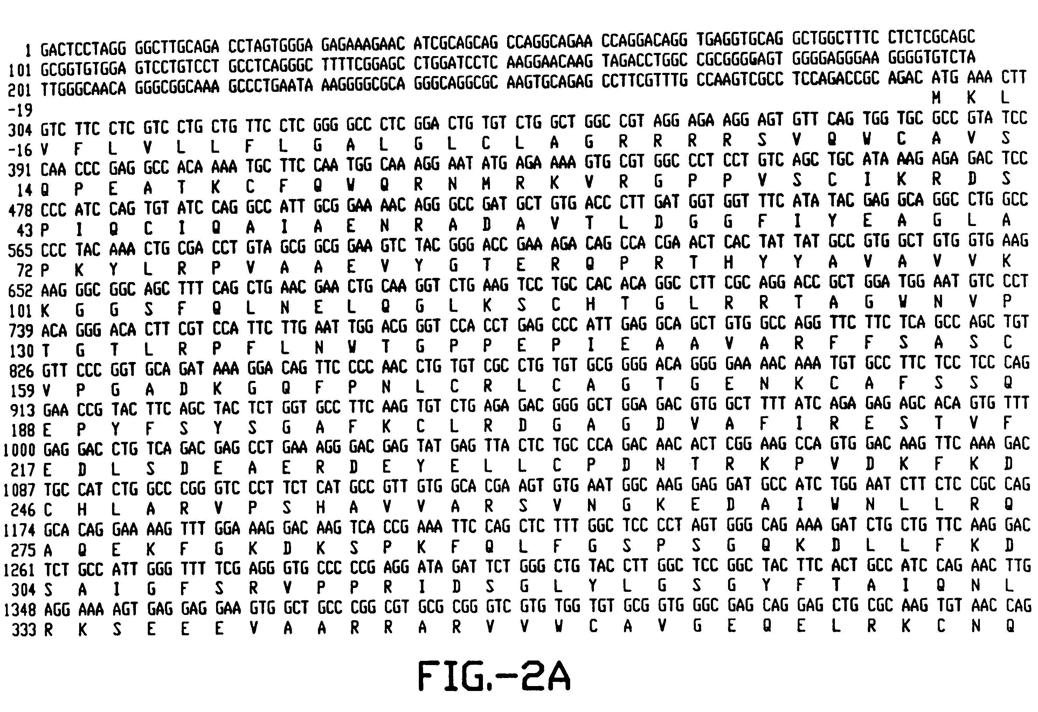

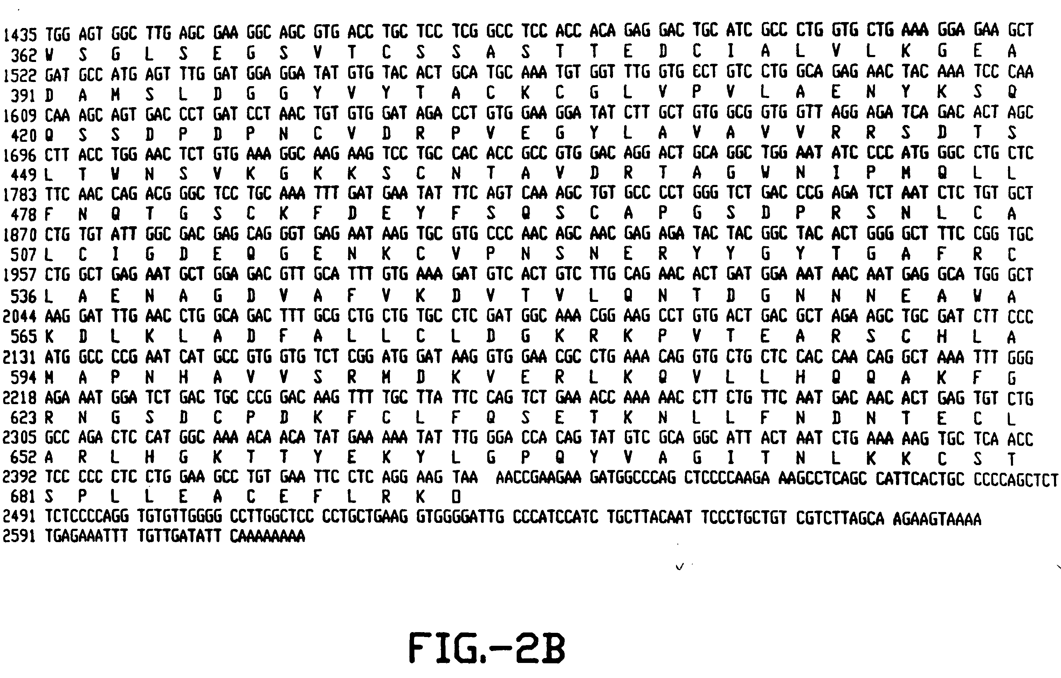

As used herein, "human lactoferrin" comprises a polypeptide having the amino acid sequence substantially as described by Metz-Boutigue, et al. (1984), Eur. J. Biochem., 1451, 659-676 and as set forth in Fig. 2. It is noted, however, that an earlier partial sequence of the human lactoferrin sequence disclosed a number of discrepancies between the published sequence and that obtained herein. Specifically, the following discrepancies exist (amino acid numbering is from the sequence in Figure 1 with DNA position in parenthesis) :

| Amino Acid | Position | In Metz-Boutique |
|---|---|---|
| Arg | 122 (418) | Absent |
| Thr | 130 (442) | Ile |
| Gln | 151 (505) | Arg |
| Ser | 184 (604) | Leu |
| Tyr | 189 (619) | Lys |
| Ser | 372 (1169) | TrP |
| between Ala and Met | 391 (1122) | 13 amino acids |
| Cys | 403 (1225) | Gly |
| Gln | 512 (1588) | Glu |
| Lys | 675 (2077) | Arg |

Accordingly, human lactoferrin is also defined by the sequence shown in Figure 1 which combines the sequence differences obtained herein with the published sequence. The term human lactoferrin also includes allelic variations of either of these sequences or recombinant human lactoferrin variants wherein one or more amino acids have been modified by the substitution, insertion or deletion of one or more amino acid residues. In some instances human lactoferrin may be produced in milk with all or part of a secretory signal sequence covalently attached thereto.

As used herein, a "human lactoferrin DNA sequence" is a DNA sequence which encodes human lactoferrin as defined above. Such a human lactoferrin DNA sequence may be obtained from a human mammary gland cDNA library or may be derived from the human genome. Example 2 herein describes the cloning and nucleotide sequence of human lactoferrin derived from a human mammary gland cDNA library. The DNA sequence of this human lactoferrin is shown in Fig. 1 and Fig. 2 and is substantially the same as that described by Rado, et al. (1987), Blood, 70, 989-993. The construction of plasmids containing an expressible transgene encoding hLF is described in the examples. One of these plasmids is cGP1HLF also sometimes referred to as 16,8HLF3) contains a transgene designed for tissue-specific expression in bovine mammary secretory cells.

In a second embodiment of the invention, transgenes are provided for producing human serum albumin in the milk of transgenic bovine species. Human serum albumin is a serum protein which contains 584 amino acid residues (Minghetti, et al. (1986), J. Biol. Chem., 261, 6747). It is the most abundant protein in human serum and performs two very important physiological functions. Serum albumin is responsible for about 80% of the total osmolarity of blood and it transports fatty acids between adipose tissues.

Human serum albumin is used primarily to expand plasma volume by restoring osmotic pressure in the circulatory system. Currently, a heat treated serum derived hSA fraction is infused in most shock and trauma victims, including most of the patients undergoing extensive surgery. HSA is presently derived from human blood plasma as a by-product from blood fractionation processes to obtain rare blood proteins such as factor VIII and IX. The recently developed technology of producing such factors by biotechnological means, however, threatens the source of human serum albumin.

As used herein "human serum albumin" comprises a polypeptide having the amino acid sequence substantially as that described by Minghetti, et al., ibid; Lawn, et al. (1981), Nucl. Acids Res., 9, 6103. Also included are variations thereof including recombinant human serum albumin variants wherein one or more amino acids have been modified by the substitution, insertion or deletion of one or more amino acid residues. (Minghetti et al. (1986) J. Biol. Chem. 261, 6747-6757.) In some instances, human serum albumin may be produced in milk by expressing a transgene which contains DNA encoding the secretory signal sequence of hSA. Alternatively, human serum albumin may be produced in and secreted from liver cells of a transgenic animal utilizing a completely heterologous transgene comprising human genomic DNA encoding 5' expression regulation sequences, the human serum albumin secretion signal and structural gene and 3' expression regulation sequences. As indicated in the Examples, transgenes containing this heterologous sequence were formed by in vivo homologous recombination of overlapping transgene fragments to reconstitute the hSA gene in the transgenic animal. The so formed transgenic animal produced human serum albumin in its circulatory system.

As used herein, a "human serum albumin DNA sequence" is a DNA sequence which encodes human serum albumin as defined above. Such a human serum albumin DNA sequence may be obtained from λHAL-HAI, λHAL-3W and λHAL-HI4 as described by Urano et al: (1986) J. Biol. Chem. 261, 3244-3251 and Urano et al. (1984) Gene 32, 255-261 and in the Examples herein.

The human serum albumin DNA sequence was cloned as described in Example 10 herein and subsequently manipulated to substitute for the human lactoferrin gene encoded in plasmid cGP1HLF (also referred to as p16,8HLF4). From this plasmid a transgene is obtained containing 16kb of the 5' expression regulation sequence of the bovine αS1-casein gene, human serum albumin DNA sequence and approximately 8kb of the 3'-flanking region of the αS1-casein bovine gene. This transgene is used to microinject fertilized oocytes from bovine species. After early detection of transgenesis, blastocysts containing the hSA transgene are implanted into a recipient female bovine species and brought to term.

The following is presented by way of example and is not to be construed as any limitation on the scope of the invention.

### EXAMPLE 1

### Construction of a probe specific for bovine αS1-casein sequences.

### A. Isolation of Chromosomal DNA

Placental tissue was obtained from the slaughterhouse. Surrounding connective tissue was removed and pieces of about 30 grams were quickly frozen in liquid N₂. Chromosomal DNA was isolated as follows: 30 grams of tissue was homogenized (on ice) with 35 ml of Buffer 1 containing 300 mM Sucrose; 60 mM KCl; 15 mM NaCl; 60 mM Tris.HCl pH 8.2; 0.5 mM spermidine; 0.15 mM spermine; 2 mM EDTA; 0.5 mM EGTA. 65 ml of icecold buffer 1 containing 1% NP40 was added and the mixture was incubated for five minutes on ice. After centrifugation for five minutes at 3000 xg the pellet was rinsed with buffer 1 containing 1% NP40. After repeating the centrifugation step the pellet was resuspended in 5 ml of buffer 1. 5 ml 0.5 M EDTA was quickly added. Final volume was now 15 ml. 0.15 ml of a 10% SDS solution was added. After mixing, RNAse A and T1 were added to final concentrations of 0.4 mg/ml and 6 u/ml respectively. After incubation at 37°C for three hours, Proteinase K was added to a final concentration of 0.1 mg/ml. This mixture was incubated for 15 hours at 37°C. The mixture was then carefully extracted with phenol. The aqueous phase was isolated and 1/30 volume of 3M NaOAc pH 5.2 and one volume of isopropylalcohol was added. The precipitate (DNA) was rinsed with 70% ethanol and slowly dissolved in 0.5 ml of 10 mM Tris.HCl pH 8.0; 1 mM EDTA, at 4°C.

### B. Amplification of Sequences from the 5'-flanking Region of the αS1-casein Gene

Two DNA-primers were synthesized based on the sequence published by Yu-Lee et al., (1986) Nucl. Acids Res. 14, 1883-1902. Primer 1 was located at position-681 relative to the major transcription initiation site and had the following sequence:

5'-TCC ATG GGG GTC ACA AAG AAC TGG AC-3'.
(Seq. ID No.: 5)

Primer #2 was located at position +164 relative to the major transcription initiation site and had the following sequence: 5'-TGA AGC TTG CTA ACA GTA TAT CAT AGG-3' (Seq. ID. No.: 6). The first eight nucleotides of this primer are not encoded by the bovine genome, but contain a HindIII restriction site to facilitate subsequent cloning steps. These primers were annealed to the chromosomal DNA and extended in the presence of deoxynucleotides by TAQ-polymerase. After three minutes the mixture was denatured for one minute at 92°C, reannealed at 50°C for 1.5 minutes and again incubated at extension temperature (68°C) for 2 minutes. This cycle was repeated 30 times. After the last cycle DNA was checked for the presence of the expected EcoRI sites. Both the size of the fragment and the presence of EcoRI sites was as expected. The fragment was then treated with Klenow enzyme to repair any overhanging ends, treated with kinase to attach phosphate groups at the ends of the fragment, incubated at 65°C for 10 minutes to inactivate the kinase and klenow enzymes and finally digested with HindIII. This fragment was then subcloned in pUC19 (Yanisch-Perron, et al. (1985), Gene, 33, 103-109) digested with SmaI and HindIII. Formal proof of the identity of this fragment was obtained by sequencing parts of this subclone (after re-cloning into M13 vector). The determined sequence was identical to the published sequence. This probe was then used to screen a bovine genomic library to obtain clones specific for the 5'-flanking region of the αS1-casein gene.

### C. Amplification of Sequences from the 3'-flanking Region of the αS1-casein Gene

A similar approach was taken as described above. Two primers were designed based on the sequence published by Stewart et al (1984) Nucl. Acids Res. 12, 3895-3907. The 5'-primer was located just downstream of the coding sequence starting at position 713 of the cDNA sequence. It had the following sequence:

5'-GAG GGA CTC CAC AGT TAT GG-3' (Seq. ID No.: 7).

The other primer was located at position 1070 of the cDNA sequence and had the following sequence: 5'-GCA CAC AAT TAT TTG ATA TG-3'(Seq. ID No.: 8). These primers were annealed to the chromosomal DNA and the region between these primers was amplified as described above. The resulting fragment was ≈900 bp longer then expected. Sequence analysis showed that an intervening sequence of this size was present between nucleotide 737 and 738 of the cDNA. The amplified fragment was treated with Klenow-polymerase to repair any overhanging ends and treated with kinase to attach phosphate groups to the ends of the fragment. The fragment was then ligated into pUC19 previously cut with SmaI.

### D. Screening of a Bovine Phage Library for αS1-casein Flanking Sequences

A bovine genomic library, constructed in EMBL3, was obtained from Dr. M. Groenen, Agricultural University Wageningen, Netherlands, and was screened in the following way. The bacteriophage particle titre was determined on Escherichia coli MB406 a permissive host strain (Stratagene Inc.). For this, several dilutions of the phage stock were made in SM buffer (50 mM Tris.HCl pH 7.5, 100 mM NaCl, 10 mM MgSO4, 0.01% gelatin) and mixed with 200 µl MB406 (O.D.₅₅₀ = 0.9); after 20 minutes at 37°C, 3 ml top agarose (Luria-Bertani medium, 0.8% agarose, 10 mM MgCl₂) was added and this was plated on LB plates and incubated overnight at 37°C.

Approximately 600,000 phages were then plated by adding the required amount of phage stock to 400 µl MB406. The subsequent plating was as described as above. The next step was transfer of the phage to nitrocellulose filters. Plates were placed at 4°C for one hour. Nitrocellulose filters (S&S) were placed on the top agarose layer and exact position was marked. After lifting, the filters were soaked for (1) 30 minutes in denaturation buffer (1.5M NaCl, 0.5M NaOH); (2) 5 minutes in neutralizing buffer (1.5M Nacl, 0.5M Tris.HCl pH 8.0). After rinsing with 2xSSPE (360 mM NaCl, 20 mM NaH₂PO₄, 2 mM EDTA), the filters were baked under vacuum at 80°C for two hours.

Prehybridization of the filters was performed in a buffer containing 50% formamide, 5x Denhardt's solution (0.1% Ficoll, 0.1% polyvinylpydrolidone, 0.1% bovine serum albumin), 5xSSPE, 0.1% SDS and 100 µg/ml denatured salmon sperm DNA at 42°C for two hours. Hybridization was performed in same buffer at 42°C overnight in a shaking waterbath. The probe, generated as previously described, was labelled using the Random Primed labelling kit from Boehringer Mannheim. After overnight hybridization the filters were washed three times with 2xSSC, 0.1% SDS at room temperature.

Overnight exposure of Kodak XAR films was performed with amplifying screens (Dupont) at -70°C. Putative positives were plugged out of the plates and put overnight in SM buffer at 4°C. These were plated out as described above and DNA was isolated following the plate lysate method (Maniatis, T., et al. (1982), Molecular Cloning: A Laboratory Manual, Cold Spring Harbor, N.Y.). 5 ml SM buffer was added to the top agarose layer; after two hours gentle shaking buffer was removed and spun at 4000 rpm at 4°C for 10 minutes. Supernatant was transferred to sterile tubes and RNase A and DNaseI (both final concentration 1µg/ml) was added, this was incubated at 37°C for 30 minutes. One volume of a 20% polyethyleneglycol, 2.5 M Nacl solution was added and put on ice for one hour. Centrifugation at 4000 rpm for 30 minutes at 4°C left precipitated bacteriophage particles. These were resuspended in 500 ml SM buffer, SDS (final concentration 0.1%) and EDTA (final concentration 5 mM) was added, this was incubated at 68°C for 15 minutes. Protein was removed with one phenol and one chloroform extraction step. Precipitation of phage DNA was performed with one volume isopropanol. Phage DNA was washed once with 70% ethanol and dissolved in 50 ml Tris.HCl pH 7.5, 1 mM EDTA buffer.

Restriction enzyme analysis, agarose gel electrophoresis, transfer of DNA from gel to nitrocellulose filter and Southern blotting were all done according to standard procedures (Maniatis (1982), Molecular Cloning: A Laboratory Manual). Hybridization with probes (described hereinafter) was performed according to the same procedure as the screening conditions described above.

### E. Isolation of Clones Containing 5'-flanking Region of Bovine S1-casein

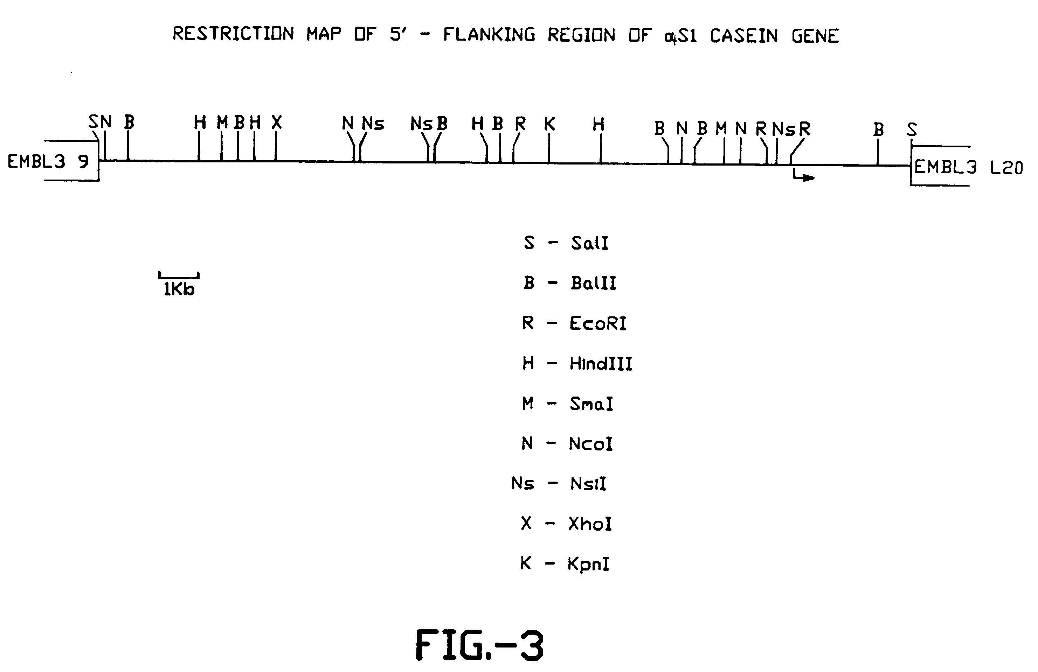

Three putative clones were identified using the probe and procedures as described above. After another round of screening, clean recombinant bacteriophage was analyzed. Digestion of cloned DNA with SalI, EcoRI and SalI/EcoRI (double digestion) and hybridization with the probe described above showed identical inserts in all three clones. The insert consisted of an 18kb (partial Sau3A fragment excised with SalI). Transciptional orientation in the clone was determined with hybridization of above described restriction fragments with (1) probe 1 described above, and (2) the NcoI-NsiI fragment of probe 1. This showed a region of about 16kb upstream of transcription start. Downstream from the transcription start was another 1.9kbp. Sequencing of part of the latter region showed the presence of exon 2 and part of intron 2 of the bovine αS1-casein gene. Additional sequencing of the region-103 - +300 confirmed the identity of the clone. The ethidium-bromide pattern of the described restriction fragments also showed the orientation of the clone in the EMBL vector. Subsequent analysis of the clone with the following restriction enzymes (NcoI, PstI, KpnI, BamHI, HindIII, BglII) resulted in the restriction map of 5' flanking region of bovine S1-casein gene as shown in Fig. 3.

### F. Isolation of Clones Containing 3'-flanking Region of Bovine αS1-casein

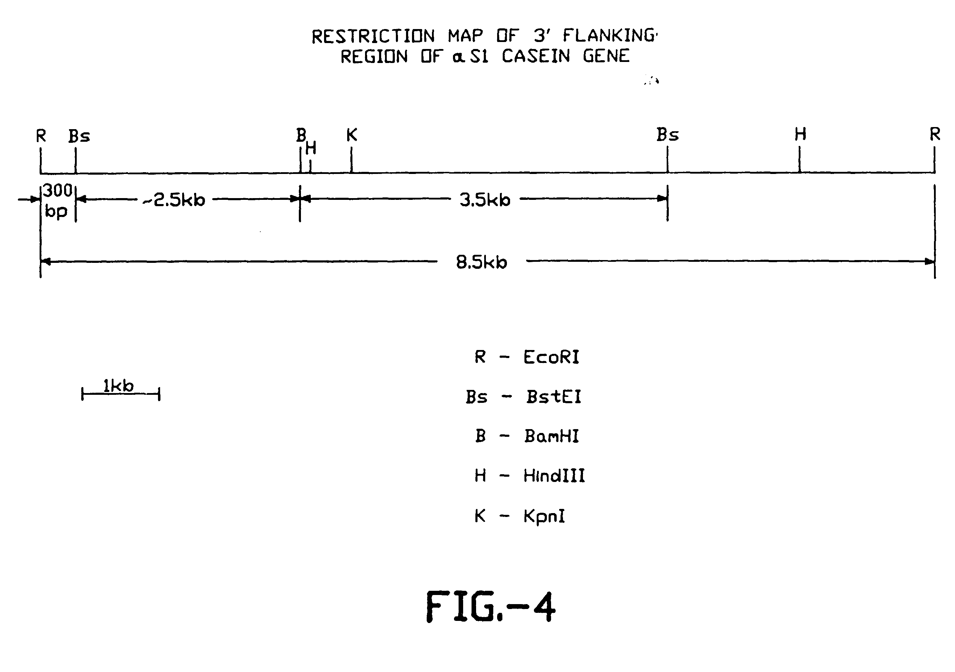

Duplicate nitrocellulose filters from the initial phage plating used for isolating 5' clones were screened with the 3' αS1-casein probe using the same hybridization conditions previously described. Eight positive clones were identified after two rounds of screening. Phage DNA was prepared as described. Subsequent restriction digests with SaII, EcoRI, and SaI/EcoRI and Southern hybridization with the 3' αS1 probe showed identical inserts in seven of the eight clones. One clone containing an 18.5kb EcoRI insert was further analyzed with the restriction enzymes BsteII and BamHI. A restriction map of that clone is shown in Fig. 4.

### EXAMPLE 2

### Cloning of Human Lactoferrin Gene

### A. Materials

Restriction endonucleases, T4 ligase, and T7 polynucleotide kinase were obtained from Boehringer-Mannheim, New England Biolabs, or Bethesda Research Laboratories. Radio-isotopes were purchased from Amersham. A human mammary gland cDNA library in bacteriophage Xgtll was obtained from Clontech, Inc., Palo Alto, Calif.

### B. Isolation of the Human Lactoferrin Gene

The human mammary gland library was screened by standard plaque hybridization technique (Maniatis, et al. (1982) Molecular Cloning: A Laboratory Manual) with three synthetic oligomers. Two of the oligomers were 30-mers corresponding to the cDNA sequence of Rado et al., supra, at amino acid positions 436-445 and 682-691. The third was a 21-mer "best guess" probe based on human codon bias and coding for amino acid sequence of HLF between amino acid residues 18 and 24. Respectively, they were:

(1) 5'-CTTGCTGTGGCGGTGGTTAGGAGATCAGAC-3' (Seq. ID
No.: 9)
(2) 5'-CTCCTGGAAGCCTGTGAATTCCTCAGGAAG-3' (Seq. ID
No.: 10), and
(3) 5'-ACCAAGTGCTTCCAGTGGCAG-3'( Seq. ID No.: 11).

The probes were radiolabeled (Crouse et al. (1983) Methods Enzymol. 101, 78-98) and used to screen duplicate filters. Filters were washed at a final stringency of 2 X SSC, 37°C.

### C. Nucleotide Sequence Analysis

DNA fragments were isolated by use of low-melting agarose (Crouse et al, supra) and subcloned into bacteriophase M13mp18 or M13mp19 (Messing et al. (1983) Methods Enzymol. 101, 20-78). The sequence was determined using the Sequenase enzyme (modified T7 DNA polymerase) (Tabor et al. (1987) Proc. Natl. Acad. Sci. USA 84, 4767-4771). All reactions were carried out according to the manufacturer's specifications (US Biochemicals). The sequence is shown in Fig. 1. The hLF sequence was digested with HindIII and EcoRI (present in the surrounding phage sequences) and subcloned into the HindIII and EcoRI site of pUC19 to form pUS119 Lacto 4.1. This clone contained the entire coding sequence of the mature form of hLF, but lacked the complete signal sequence.

### EXAMPLE 3

### Construction of bovine αS1-casein CAT vectors

In order to determine whether the αS1-casein fragments obtained in Example 1 had promoter and other properties needed to express a heterologous gene, expression plasmids were constructed containing variable amounts of 5-' and 3'-flanking regions from the αS1-casein gene. The chloramphenicol Acetyl transferase gene (CAT) was used as a heterologous gene in these vector constructs. The CAT gene is useful to detect the expression level for a heterologous gene construct since it is not normally present in mammalian cells and confers a readily detectable enzymatic activity (see Gorman, C.N., et al. (1983), Mol. Cell. Biol., 2, 1044-1051) which can be quantified in the cells or animals containing an expressible gene.

### A. DNA sequences.

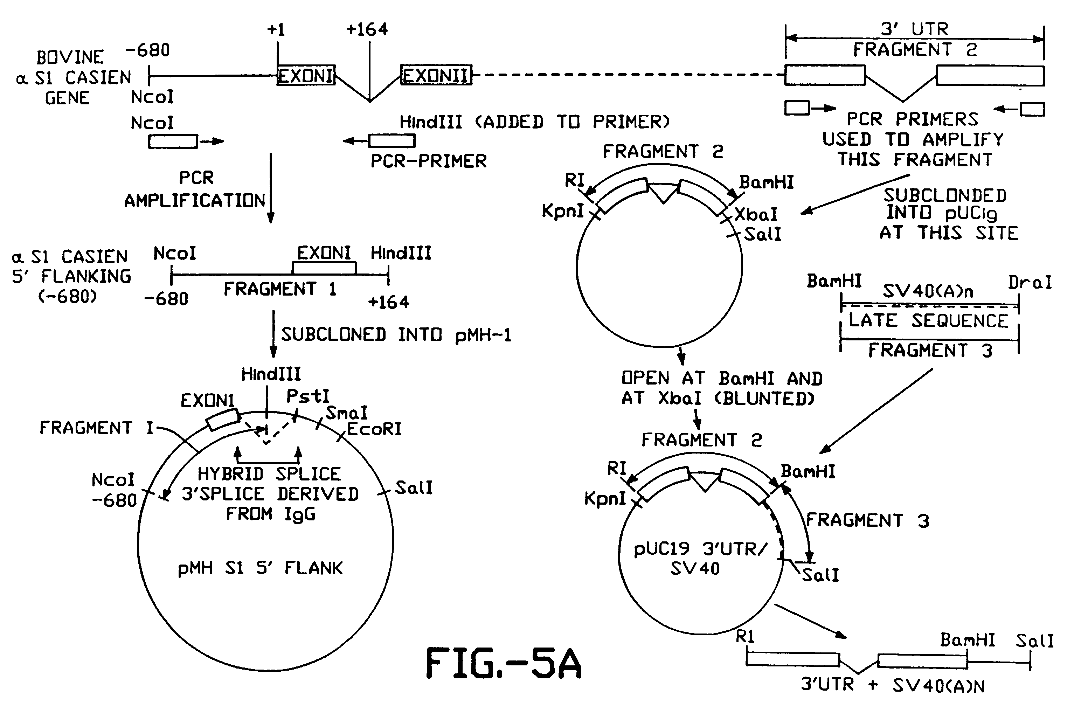

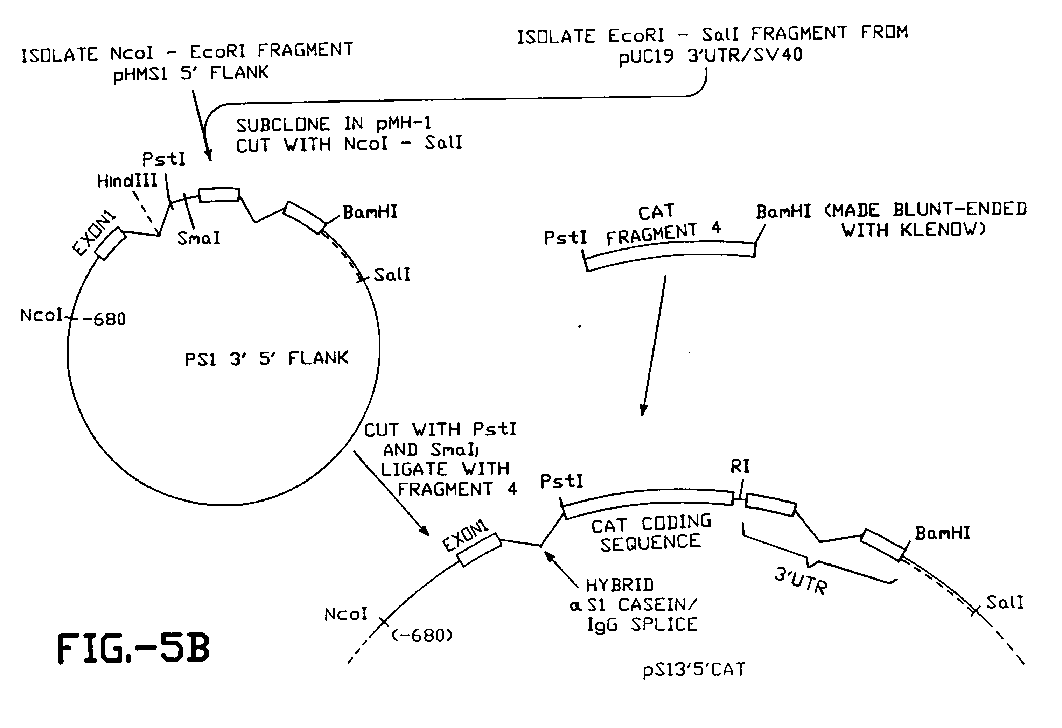

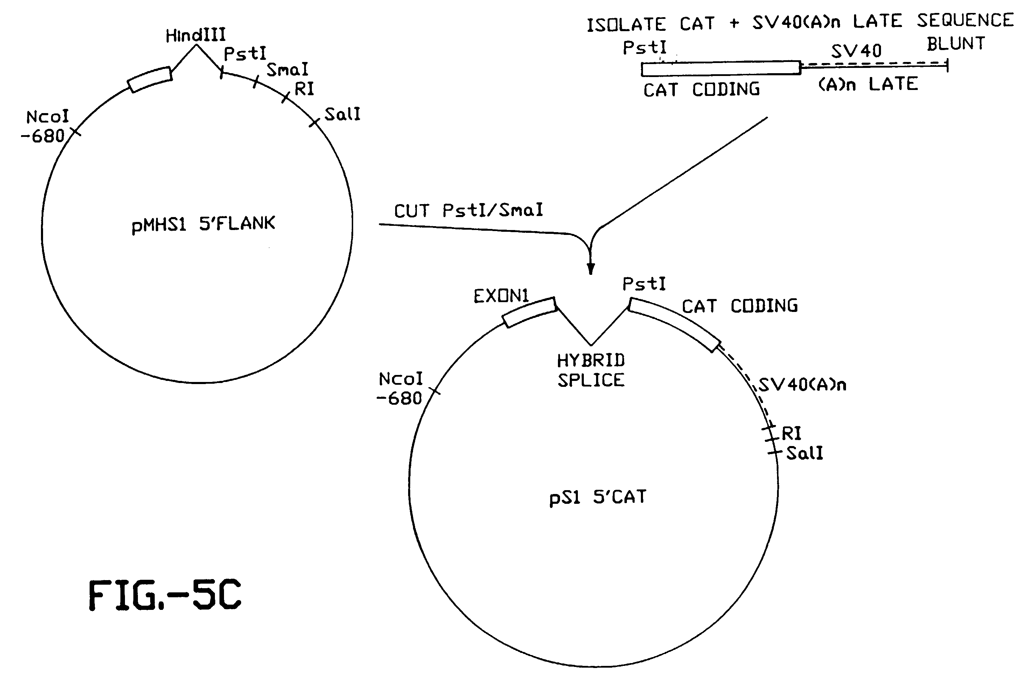

681 bp of a αS1-casein promoter plus the first noncoding exon plus approximately 150 bp of the first intervening sequence (IVS) were isolated from a 5'-flanking genomic clone from Example 1 by PCR amplification as an NcoI-HindIII fragment (approximately 830 bp). This fragment is identified as fragment 1 in Fig. 5A. The primer sequences consisted of: 
5'-TCCATGGGGGTCACAAAGAACTGGAC-3'
(Seq. ID No.: 12) and
5'-TGAAGCTTGCTAACAGTATATCATAGG-3'
(Seq. ID No.: 13)
 that were designed from a sequence published by Yu-Lee et al. (1986) Nuc. Acids Res. 14, 1883-1902.

Approximately 1.6kb (fragment 2, Fig. 5A) of αS1-casein 3'-flanking sequence was isolated by PCR amplification from a bovine 3'-flanking genomic clone from Example 1. This region contained the previously described splice within the 3' untranslated region of αS1-casein gene. Fragment 2 was subcloned into the SmaI site of pUC19. The primer sequences consisted of: 
5'-GAGGGACTCCACAGTTATGG-3'
(Seq. ID No.: 14) and
5'-GCACACAATTATTTGATATG-3'
(Seq. ID No.: 15)
 that were designed from a sequence published by Stewart et al. (1984) Nucl.Acids Res. 12, 3895-3907.

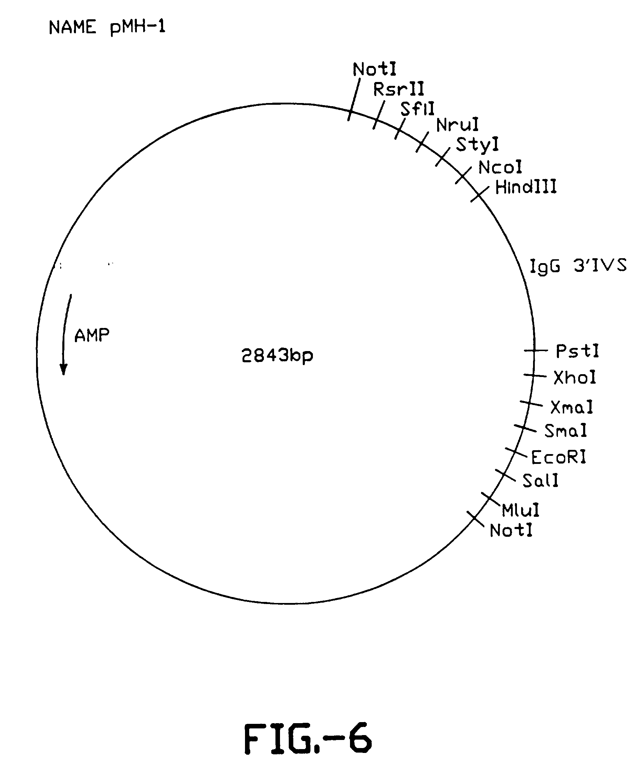

A hybrid splicing signal comprising the 3' splice site of an immunoglobulin gene (Bothwell et al. (1981), Cell, 24, 625-637) was synthetically prepared and inserted into pUC18 along with unique restriction sites flanking either side to produce pMH-1. This plasmid is shown in Fig. 6. NcoI and HindIII sites were designed such that ligation with fragment 1 from the bovine 5' genomic clone would result in the functional hybrid splice sequence. See Fig. 11.

A polyadenylation sequence was obtained from SV40 virus as a BamHI-DraI fragment (fragment 3 in Fig. 5A) isolated from pRSVcat (Gorman, C.M., et al. (1982), Proc. Natl. Acad. Sci., 79, 6777-6781).

A bacterial CAT coding sequence was subcloned into pUC19 as a PstI-BamHI fragment.

### B. Construction of pS13'5'CAT

Fragment 1 of αS1-casein promoter was subcloned into pMH-1 (Fig. 6) between the NcoI and HindIII sites to form pMHS15'flank.

The SV40 polyadenylation sequence (fragment 3) was subcloned as a BamHI-DraI fragment into pUC19 immediately 3' to the 3' αS1-casein flanking sequence (fragment 2) to form pUC19 3' UTR/SV40. This allowed for the removal of a continuous EcoRI-SalI fragment (containing the 3'-flanking sequence and poly (A) sequence) that was subcloned into pMH-1 to derive pMHS13'UTR (Fig. 5B) which was used later to construct pMHSI 3' UTR hlf which contains sequences encoding human lactoferrin.

The EcoRI-SalI sequence (fragments 2 and 3) were subcloned into the EcoRI-SalI sites of pMHS15'flank to form pS13'5'flank.

The PstI-BamHI CAT fragment (fragment 4 in Fig. 5B), after blunting the BamHI site with Klenow, was subcloned into pS13'5'flank (Fig. 5B) between the PstI and SmaI sites to form pS13'5'CAT.

### C. Construction of pS15'CAT

The CAT fragment (fragment 4 in Fig. 5B, PstI-BamHI) and SV40 polyadenylation fragment (fragment 3 in Fig. 5A, BamHI-DraI) were subcloned into the PstI and SmaI sites of pHHSl5'flank to form pS15'CAT (Fig. 5C).

### D. Assay for CAT Production

Each of these CAT plasmids were transfected into human 293S cells (Graham, F.L., et al. (1977), J. Gen. Virol., 36, 59-72) by the calcium phosphate co-precipitation method (Gorman, C.M., et al. (1983), Science, 221, 551; Graham, F.L., et al. (1973), Virology, 52, 456-467). Cells were harvested 44 hours after transfection and cell extracts were assayed for CAT activity (Gorman, C.M., et al. (1982), Mol. Cell. Biol., 2, 1011; deCrombrugghe, B., et al. (1973), Nature [London], 241, 237-251, as modified by Nordeen, S.K., et al. (1987), DNA, 6, 173-178). A control plasmid expressing CAT driven by the Cytomegalovirus Immediate early promoter (Boshart, M., et al. (1985), Cell, 41, 521) was transfected into human 293 S cells to assay for transfected efficiency.

pS13'5'CAT was expressed in these cells at a level which was approximately 30-100 fold lower than the control plasmid, but significantly higher than background. Primer extension analysis indicated that transcription had initiated predominantly in the expected region.

When pS15'CAT was transfected into 293S cells, expression was also detected.

### EXAMPLE 4

### Bovine αS1-casein/human lactoferrin expression cosmid cGP1HLF

### A. Construction of DNA Sequences.

16kb of bovine αS1-casein 5'-flanking sequence from Example 1 was isolated from the bovine genomic library (phage GP1) as a SalI-BglII fragment. The BglII site lies at the junction of the first intron and second exon of the αS1-casein gene.

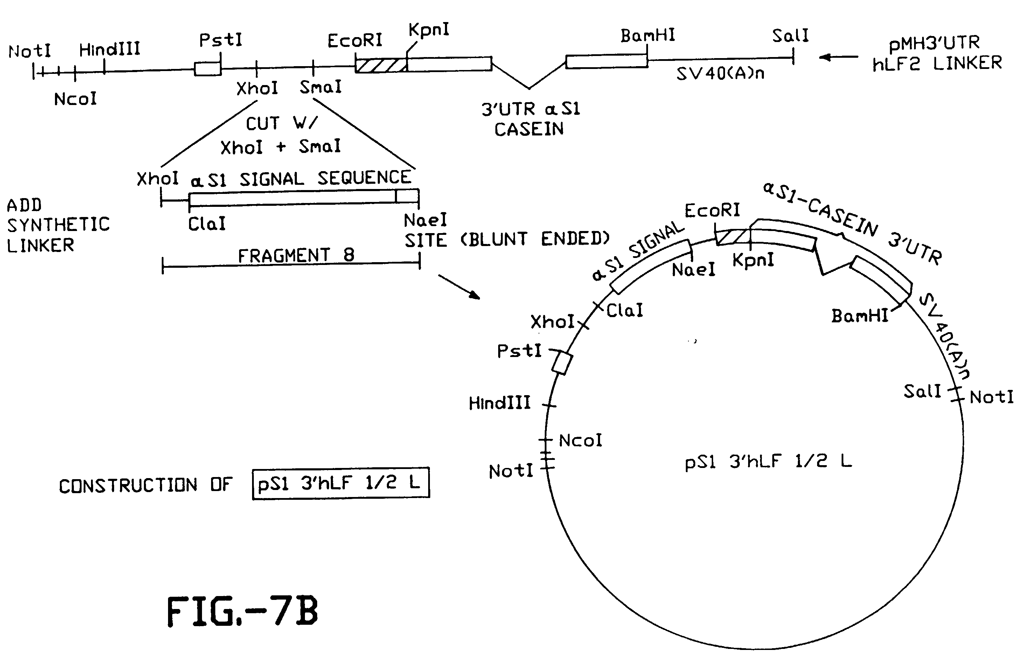

Bovine αS1-casein signal sequence (Stewart et al. (1984) Nucl. Acids Res. 12, 3895) was prepared from synthetic DNA synthesized on a Cylone Plus® DNA Synthesizer (Millgen/Biosearch I) and contained the entire signal sequence plus XhoI and Cla I sites attached to the 5'-end, and NaeI to the 3'-end (fragment 8, Fig. 7B).

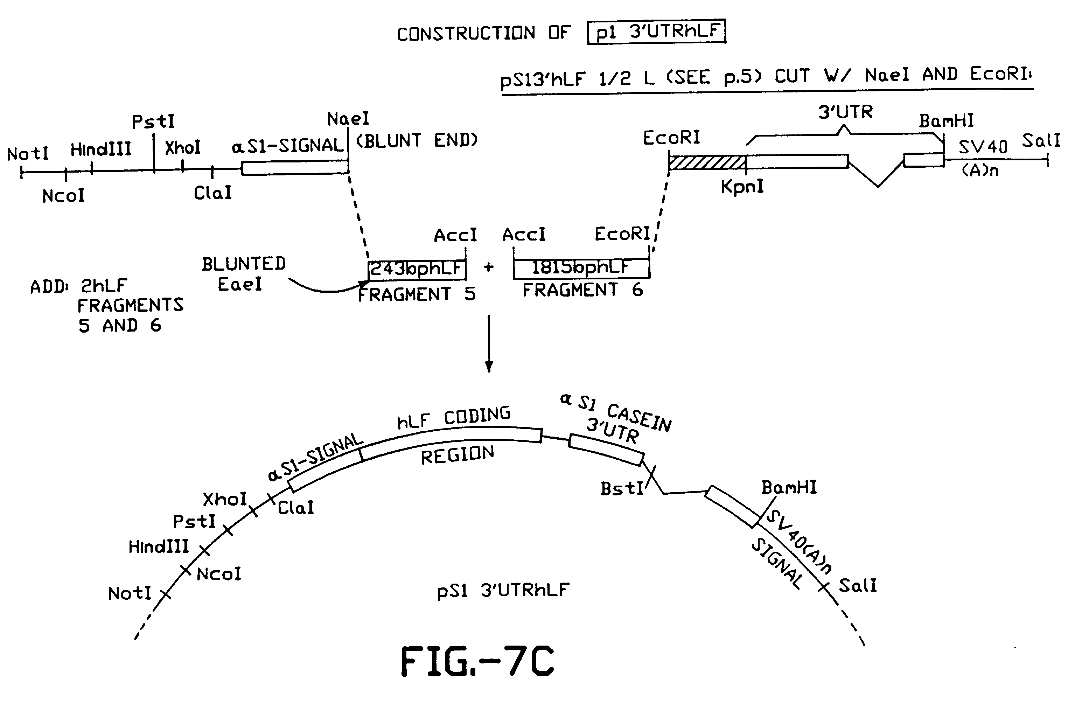

Cleavage of pUC119 Lacto 4.1 with EaeI precisely opened the plasmid at the codon for the first amino acid of mature hLF. Treatment with Klenow was used to fill in the overhanging 5'-end. Further digestion with AccI and EcoRI gave two fragments: (a) an EaeI-AccI fragment containing the first 243 bp of mature hLF (fragment 5, Fig. 7C), and (b) a contiguous AccI-EcoRI fragment (fragment 6, Fig. 7C) of 1815 bp that contained all but five terminal codons of the remaining coding sequence.

A synthetic linker was prepared that contained the last five codons of hLF beginning at the EcoRI site and extending for four bases beyond the stop codon. A KpnI site was added to the 3'-end (fragment 7 in Fig. 7C).

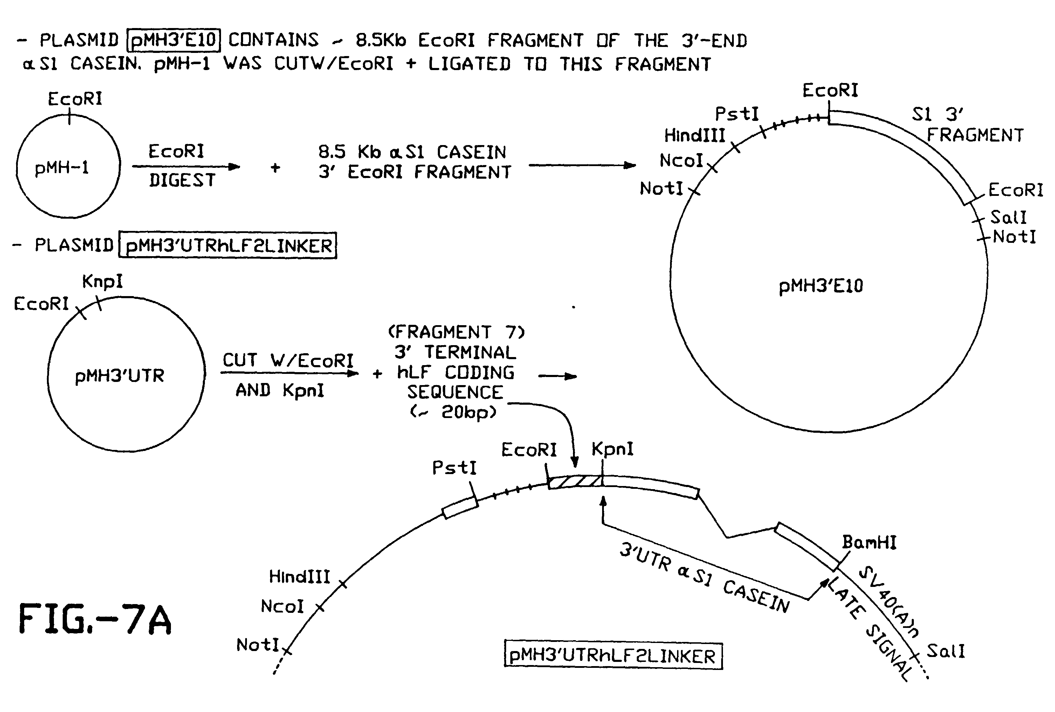

An 8.5kb EcoRI 3'-fragment was isolated from the bovine genomic library (Fig. 4) containing sequences beginning just downstream of the coding region of αS1-casein and a BstEII site approximately 350 bp from the 5'-end. This fragment was subcloned into pMH-1 at the EcoRI site to form pMH3'E10 (Fig. 7A). A SalI site is adjacent to the 3'-EcoRI site in pMH3'E10.

### B. Construction of cGP1HLF

The hLF 3'-linker (fragment 7, Fig. 7C) was subcloned into the EcoRI-KpnI sites of pMH3'UTR (Fig. 7A) to produce pMH3'UTRhLF2linker (Fig. 7A).

The synthetic bovine αS1-casein signal sequence (fragment 8) was then subcloned into the XhoI and SmaI sites of pMH3'UTRhLF2linker to make pS13'hLF1/2L (Fig. 7B).

The two hLF coding fragments (fragments 5 and 6 in Fig. 7C) were subcloned into the NaeI and EcoRI sites of pS13'hLF1/2L (Fig. 7B) to make pS13'UTRhLF (Fig. 7C).

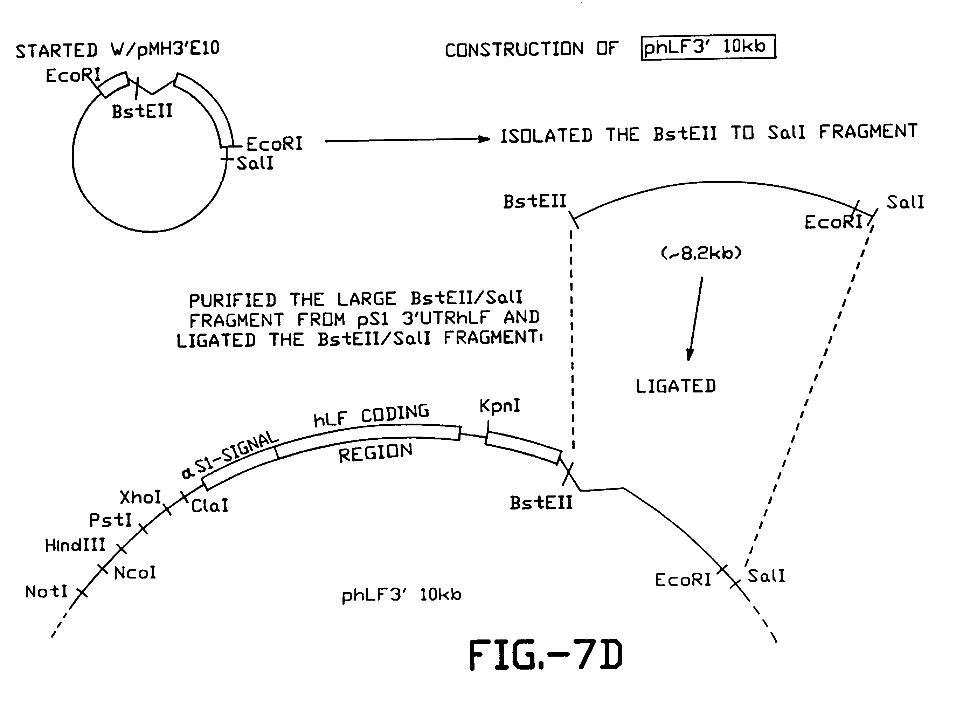

The large αS1-casein 3'UTR fragment from pMH3'E10 (Fig. 7A) was isolated as a BstEII-Sa1I fragment and subcloned into the same sites of pS13'UTRhLF to form phLF3'10kb (Fig. 7D).

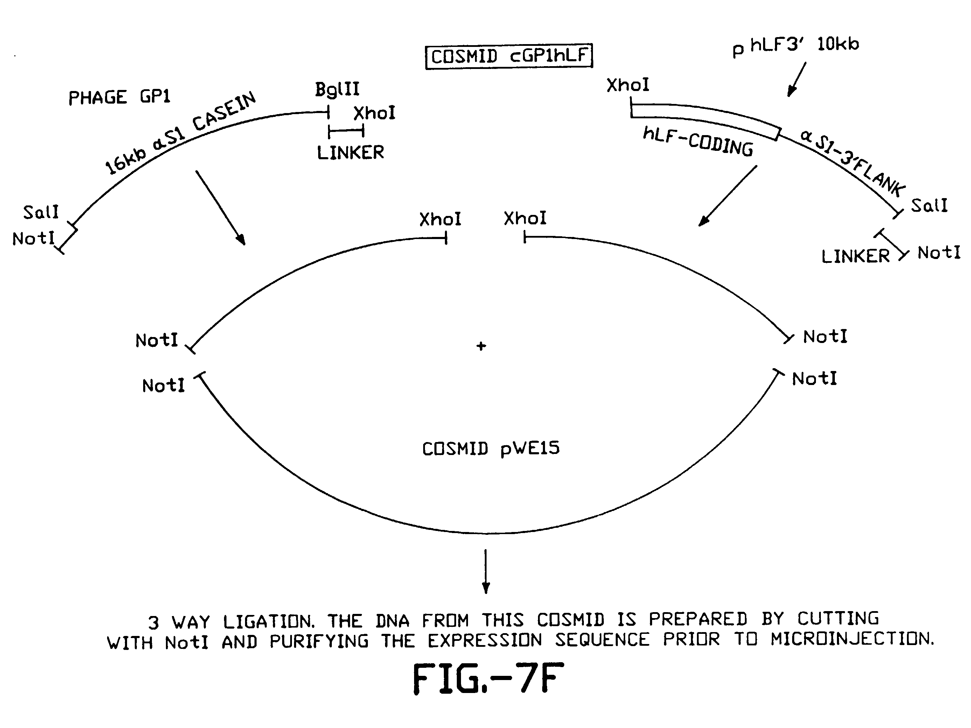

Cosmid cGP1HLF was prepared from a 3-way ligation (Fig. 7F):
(1) the 16kb 5'-flanking sequence from phage GP1 (Example 1, Fig. 3) was modified by attaching two linker adapters. The SalI site at the 5'-end was ligated to a NotI-SalI linker. The BglII site at the 3'-end was ligated to a BglII-XhoI linker;
(2) the hLF coding region, flanked on the 5'-end by the αS1-casein signal sequence and on the 3'-end by approximately 8.5kb of αS1-casein 3'-flanking sequence, was isolated as a XhoI-Sa1I fragment from phLF3'10kb. The SalI site at the 5'-end was ligated to a SalI-NotI linker;
(c) Cosmid pWE15 (Stratagene, Inc.) was linearized with NotI.

Fragments from (a), (b), and (c) were ligated together and transfected into bacteria using commercial lambda packaging extracts (Stratagene, Inc.) to produce cGP1HLF.

### EXAMPLE 5

### Bovine αS1-casein/hLF expression plasmids.

### A. Construction of pS13'5'hLF

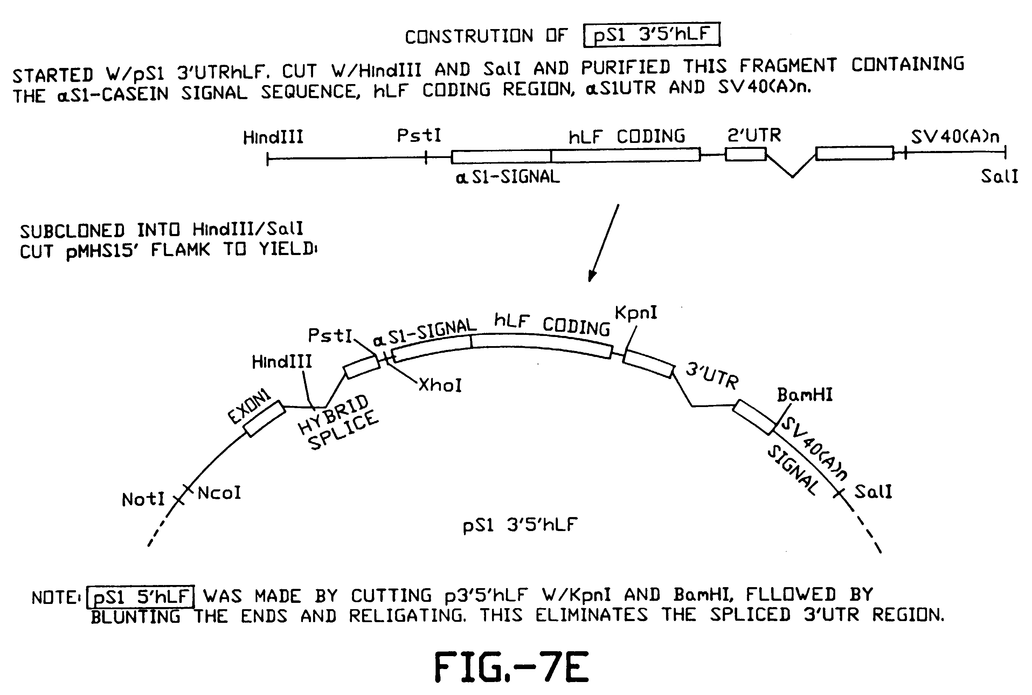

The HindIII-SalI fragment of pS13'UTRhLF was subcloned into the same sites in pMHS15'flank to form pS13'5'hLF (Fig. 7E). This plasmid contains 681 bp of bovine αS1-casein promoter sequence, the αS1-casein/IgG hybrid intron, the αS1-casein signal sequence, the hLF coding region, approximately 1.6kb of αS1-casein 3'-flanking sequence, and the SV40 late region polyadenylation sequence.

### B. pS15'hLF

Plasmid pS13'5'hLF (Fig. 7E) was cut with KpnI and BamHI which border the αS1-casein 1.6kb 3'-flanking sequence. The larger vector fragment was purified, made blunt ended with Klenow, and self-ligated to form pS15'hLF.

### C. Radioimmunoassay for hLF

An immunoglobulin-enriched fraction of ascites fluid of a monoclonal antibody against human lactoferrin, which does not cross-react with the bovine or murine protein, was prepared by 50% ammonium sulfate precipitation and coupled to CNBr-activated Sepharose 4B ( 20 mg of protein to 1 g of Sepharose). The Sepharose beads were suspended (2 mg/ml) in phosphate-buffered saline (PBS; 10 mM sodium phosphate, 0.14 M NaCl containing 10 mM EDTA, 0.1% (^{w}/v) Polylorene and 0.02% (^{w}/v) NaN₃, pH 7.4. Sepharose suspensions (0.3 ml) were incubated for five hours at room temperature by head-over-head rotation with samples (usually 50µl) in 2-ml polystyrene tubes. Sepharose beads were then washed with saline (five times with 1.5 ml) and incubated for 16 hours at room temperature with 50µl (1kBq) of ¹²⁵I-labeled-affinity-purified polyclonal rabbit anti human lactoferrin antibodies, together with 0.5 ml of PBS, 0.1% (^{w}/v) Tween-20. Thereafter the Sepharose was washed again with saline (four times with 1.5 ml) and bound radio activity was measured. Results were expressed as percent binding of the labelled antibodies added. Levels of lactoferrin in test samples were expressed in nanomolar, using purified human milk lactoferrin as a standard (serial dilutions in PBS, 10 mM EDTA, 0.1% (^{w}/v) Tween-20.

Repeated testing of standard on separate occasions revealed that this RiA was highly reproducible, intra- and inter assay coefficients of variation ranged from 5-10%. As little as 0.1 nanogram human lactoferrin is easily detected by this RIA.

### D. Expression in 293S cells

293S cells were transfected with the above hLF plasmids as described (1µg of a CMV-CAT plasmid was co-transfected as control for transfection efficiency). Forty-four hours after transfection medium was removed from the cells and assayed for hLF as described supra, RNA was isolated as described by Stryker, et al. (1989) EMBO J. 8, 2669. The results can be summarized as follows:
1. Transfection efficiencies are identical for the two hLF plasmids;
2. hLF is expressed in the cells and secreted into the medium. In both cases, the levels are about 0.4µg/ml medium using about 3x 10⁶ cells
3. The proteins behave identical to hLF in a human milk sample in a dose response assaymeasuring the amount of ¹²⁵I- anti-lactoferrin bound as a function of the amount of sample used.
4. The protein has about the same size (^{~}80kD) as in a human milk sample as judged by Western blotting.
5. The hLF RNA produced in the cells has the correct size and its level is similar for both plasmids as judged by Northern - blotting.

These data indicate that these two expression plasmids are able to express hLF. By all standards used so far, the protein is identical to hLF present in human milk. The heterologous signal sequence is functional in that it promotes secretion of the protein from the cells into the medium. Further, the casein regulatory sequences used in these plasmids are able to promote expression of a heterologous gene.

### EXAMPLE 6

### In vitro Maturation, Fertilization and Culture of Bovine Oocytes

Immature oocytes are obtained in large quantity (400-600/day) by aspirating follicles of ovaries obtained at abbatoirs. Immature oocytes are cultured for a period in vitro before they are competent to be fertilized. Once "matured", oocytes are fertilized with sperm which has also been matured, or "capacitated" in vitro. The pronuclei of the fertilized oocyte is then injected with the transgene encoding for the expression and secretion of human lactoferrin. Zygotes resulting from this in vitro fertilization and microinjection are then cultured to the late morula or blastocyst stage (5-6 days) in medium prepared, or "conditioned" by oviductal tissue. Blastocysts are then transferred non-surgically to recipient cattle for the balance of gestation or analyzed for integration of the transgene as described herein.

In vitro maturation (IVM). Ovaries are obtained immediately after slaughter at local abbatoirs and oocytes are recovered. Alternatively, oocytes are obtained from living cattle by surgical, endoscopic, or transvaginal ultrasonic approaches. In all cases, oocytes are aspirated from ovarian follicles (2-10 mm diameter). After washing, oocytes are placed in a maturation medium consisting of M199 supplemented with 10% fetal calf serum, and incubated for 24 hours at 39°C. Sirard et al. (1988) Biol. Reprod. 39, 546-552.

In vitro fertilization (IVF). Matured oocytes are fertilized with either fresh or thawed sperm. Sperm are prepared for fertilization by first obtaining a population of sperm enriched for motility by a "swim-up" separation technique (Parrish et al. (1986) Theriogenology 25, 591-600). Motil sperm are then added to a fertilization media, consisting of a modified Tyrode's solution (Parrish et al. (1986) supra.) supplemented with heparin to induce sperm capacitation (Parrish et al. (1988) Biol. Reprod. 38, 1171-1180). Capacitation constitutes the final sperm maturation process which is essential for fertilization. Sperm and oocytes are co-cultured for 18 hours. A useful feature of this IVF method is that (in the case of frozen sperm) consistent, repeatable results are obtained once optimal fertilization conditions for a particular ejaculate have been defined (Parrish et al. (1986) supra.).

In vitro culture (IVC). Conventional culture systems, which support development of murine, rabbit, or human ova, do not support development of bovine embryos past the 8-16 cell stage. This problem has been overcome by pre-conditioning culture media with oviductal tissue. Oviduct-conditioned medium will support bovine embryos past the 8-16 cell stage to the blastocyst stage in vitro (Eyestone and First (1989) J. Reprod. Fert. 85, 715-720).

Bovine embryos have proved refractory to in vitro culture. This in part stems from the existence of a "block" to cleavage in vitro at the 8-16 cell stage. This block may be alleviated by culturing embryos in the oviducts of rabbits (reviewed by Boland (1984) Theriogenology 21, 126-137) or sheep (Willadeen (1982) in: Mammalian Egg Transfer, (E. Adams, ed., pp. 185-210)); Eyestone et al. (1987) Theriogenology 28, 1-7). However, these in vivo alternatives have been less than ideal, in that: (1) they require the maintenance of large numbers of recipient animals, (2) they require surgery to gain access to the oviducts for transfer, and a second surgery (or sacrifice) to recover the embryos, (3) all transferred embryos are seldom recovered, and (4) access to embryos during culture for observation or treatment is entirely precluded. The lack of in vitro culture systems has hampered the development of various manipulation techniques (such as gene transfer by pronuclear injection) by preventing accumulation of basic information of the chronology and ontogeny of bovine development, and by complicating the process of culturing embryos to a stage compatible with non-surgical embryo transfer and cryopreservation techniques (e.g., late blastocyst stages).

Bovine embryos did not yield to attempts to culture them in vitro past the 8-16 cell "block" until Camous et al. (1984) J. Reprod. Fert. 72, 479-485 demonstrated cleavage to 216 cells when embryos were co-cultured with trophoblastic tissue.

The co-culture procedure was extended to oviductal tissue, based on the ability of homo- or hetero-oviducts to support development from zygote to blastocyst. Thus, bovine embryos co-cultured with oviductal tissue, or in medium conditioned by oviductal tissue, developed from zygote to blastocyst in vitro (Eyestone and First, (1989) J. Reprod. Fert. 85, 715-720; Eyestone W.H. (1989) "Factors affecting the development of early bovine embryos in vivo and in vitro." Ph.D. Thesis, University of Wisconsin). Blastocysts have been produced in this system after superovulation and artificial insemination, or by in vitro maturation (IVM), and fertilization (IVF) of immature oocytes. Blastocysts produced in this fashion resulted in pregnancies and live calves after transfer to recipient animals. The results obtained were as follows:

| Step | Efficiency (%) | Number (per 100) |
|---|---|---|
| IVM | 90 | 90 |
| IVF | 80 | 72 |
| IVC | 30 | 22 |
| Embryo transfer (% pregnant) | 50 | 11 |

Therefore, from an initial daily harvest of 500 oocytes, it is expected the approximately 55 pregnancies will result.

### Preparation of Oviduct Tissue Co-Culture and Conditioned Medium

1. Obtain bovine oviducts after slaughter or by salpingectomy.
2. Harvest lumenal tissue by scraping intact oviduct gently with a glass slide.
3. Wash tissue 5 times in 10 ml modified tyrodes-hepes solution (Parrish et al. (1988) Biol. Reprod. 38, 1171-1180).
4. Resuspend final tissue pellet in M199 + 10% fetal calf serum at a ratio of 1 volume tissue:50 volumes of media.
5. Tissue suspension can be used for embryo-co-culture.
6. Alternatively, media may be conditioned for 48h; after centrifuging the suspension, the supernatant may be used as embryo culture medium. Conditioned medium may be stored at -70°C, if desired. Conditioned medium should be used at full strength for embryo culture (no dilution) (Eyestone (1989) ibid).

### EXAMPLE 7

### Microinjection of hLF Transgene into Bovine Pronuclei

The DNA fragment containing the hLF expression unit is excised from the vector by digestion with the appropriate restriction enzyme(s) and separated on agarose gels. The fragment is purified by electroelution, phenol and chloroform extraction and ethanol precipitation (Maniatis et al.). The DNA fragment is dissolved in and dialyzed in 10 mM tris, 0.1 mM EDTA pH 7.2 at a concentration of 1 to 2µg/ml. Microinjection needles are filled with the dialyzed DNA solution.

Before in vitro fertilization, cumulus cells are removed from the egg by either vortexing at maximal speed for 2 minutes or pipetting the eggs up and down several times in a standard micropipet. Bovine pronuclei are injected in principle as murine pronuclei (Hogan, B. et al. (1986) in: Manipulating the mouse embryo, Cold Spring Harbor Laboratory) with an additional centrifugation step in order to visualize the pronuclei.

The injection takes place 18-24 hours after fertilization. The time varies depending on the bull used as a source of semen. Different batches of semen cause the nuclei to become visible at different times.

Bovine oocytes, matured and fertilized in vitro, are spun in an eppendorf tube in 1 ml of tyrodes-hepes solution (Parrish (1987)) at 14500 g for eight minutes (Wall et al. (1985) Biol. Reprod. 32, 645-651). The embryos are transferred to a drop of tyrodes-hepes solution on a microscope slide covered with paraffin oil. Using a hydraulic system the oocytes are fixed to the egg holder in such a way that both the pronuclei are visible (using interference-contrast or phase contrast optics). If necessary, the oocytes are rolled to change their position on the egg holder to visualize the pronuclei. The injection needle is brought into the same sharp focus of one of the pronuclei. The needle is then advanced through the zona pellucida, cytoplasm into the pronucleus. A small volume of 1-3 pl is injected (containing 20-100 DNA copies) into the pronucleus either by using a constant flow or a pulse flow (using a switch) of DNA solution out of the needle. Alternatively, two cell stage embryos are spun as described and the nuclei of both blastomers are injected as described. The injected embryos are then transferred to a drop of co-culture medium as described in Example 6 in order to develop to the morula or blastocyst stage.

### EXAMPLE 8

### Early Detection of Transgenesis with hLF Transgene

Upon the microinjection of a construct, the oocyte is cultured. A proper site of each embryo is cleaved and subjected to lysis (King, D. et al. (1988) Molecular Reproduction and Development 1, 57-62), proteolysis (Higuchi, R., (1989) "Amplifications (A forum for PCR Users." 2, 1-3) and DPNI digestion. PCR is performed as described previously (Ninomiy, T. et al. (1979) Molecular Reprod. and Devel. 1, 242-248) with sets of two primers, one in αS1 and the other in hLF cDNA sequence. For example, in a PCR where the forward primer (30mer) αS1 sequence is 
ATG AAA CTT ATC CTC ACC TGT CTT GTG
(Seq. ID No.: 16)
 and the reverse primer (30mer) in hLF sequence is GGG TTT TCG AGG GTG CCC CCG AGG ATG GAT (Seq. ID No.: 17); 971-1000 of Figure 1), a 990 bp fragment will be generated. This fragment contains the hitherto inactivated DpNI site by loss of adenosine-methylation, at 934 bp away from the start of the forward primer.

### EXAMPLE 9

### Production of hLF in Milk of Bovine Species

Bovine morula developed from microinjected oocytes are split according to the method of Donahue (Donahue, S. (1986) Genetic Engineering of Animals, ed. J. Warren Evans et al., Plenum). One half of the morula is kept in culture to develop into blastocysts. The other half is subjected to the DNA analysis as described in Example 8. When the result of this analysis is known, the morula kept in culture are developed into a blastocyst or as a source for nuclear transfer into enucleated zygotes. Blastocyst transfer into synchronized cows is performed according to the method of Betteridge (Betteridge, K.J. (1977) in: Embryo transfer in farm animals: a review of techniques and applications).

hLF is detected in the milk of lactating transgenic offspring using the RIA of Example 5.

### EXAMPLE 10

### Bovine αS1-casein/hSA Expression Plasmids

Three overlapping phage clones that contain the complete hSA gene are used to construct an expression vector for hSA. They are designated λHAL-HA1, λHAL-3W and λHAL-H14. They are described in Urano, et al. (1986), J. Biol. Chem., 261, 3244-3251; and Urano, et al. (1984), Gene, 32, 255-261. The sequence of the gene plus some surrounding regions is published in Minghetti, et al. (1986), J. Biol. Chem., 261, 6747-6757. A single phage containing the complete hSA gene is constructed as follows:

Clone HA-1 is cut with BstEII and AhaII. The ≈1400 bp fragment running from position 1784 (in the first exon, just downstream of the ATG) to 3181 is isolated and a synthetic linker is attached to the BstEII site at the 5' end containing the first few amino acids that are cut off with BstEII as well as the sequence surrounding the ATG as well as a few convenient restriction sites. This fragment is called fragment #1.

Clone 3W is cut with AhaII and SacI the ≈13.1kb fragment running from position 3181 to 16322 is isolated and a synthetic linker is attached to the SacI site to facilitate cloning in phage EMBL3. This fragment is called fragment #2.

These two fragments are ligated and cloned in phage EMBL3. After identification of the correct phage, a fragment running from just upstream of the BstEII site (where unique restriction sites have been introduced) to the SacI site are isolated and ligated from a SacI to SalI fragment (running from position 16322 to ≈21200 isolated from clone H-14. These two fragments are then ligated and cloned in EMBL4.

After cutting with ClaI (just upstream of the BstEII site, newly introduced) and BamHI (just downstream of the SalI site in the phage DNA) this new clone yields a fragment containing the complete hSA gene with about 2.5kb 3'-flanking sequence.

To construct an expression vector for hSA cosmid cGP1HLF is partially digested with ClaI and BamHI. This removes the signal sequence, the coding sequence of hLF, the 3'-UTR and poly(A) addition region of αS1-casein as well as a small region 3' of the casein gene.

This is ligated to the hSA fragment described above and the resulting cosmid is called cGP1HSA.

The expression vector so formed contains, (1) 16kb of promoter sequences derived from the αS1-casein gene, (2) the first exon and intervening sequence of this gene both present in GP1, (3) the signal sequence of the hSA gene the complete genomic gene coding for hSA including 2.5kb downstream of that gene, and (4) ≈8kb of 3'-flanking sequence derived from the αS1-casein gene.

This transgene is used to produce transgenic bovine species producing hSA in their milk in a manner analogous to that used to produce hLF in the milk of bovine species.

### EXAMPLE 11

### Purification of HSA from the Milk of Bovine Species

Purification of heterologous proteins from milk is facilitated by the fact that, following casein precipitation, those proteins, for the most part, are found in the whey fraction which is less contaminated than the production media used in microbial or cell-based systems.

Chromatographic techniques are preferred for the purification of hSA from cow milk. This approach produces a better recovery and higher albumin purity as well as a lower content of albumin polymers as compared with ethanol fractionation (Curling (1980) in: "Methods of Plasma Protein Fractionation", Curling, ed., Academic Press London, UK; Curling et al. (1982) J. Parenteral Sci. Technol. 36, 59; Berglof et al. and Martinache et al. (1982) Joint Meeting IHS-ISBT, Budapest). The specific transport role of hSA as well as its major role in maintaining intravascular osmotic pressure may also be better preserved upon chromatographic purification (Steinbruch (1982), Joint Meeting ISH-ISBT, Budapest).

The following steps are used to recover hSA produced in the milk of transgenic cows:
1. Precipitation of caseins (about 80% of milk protein) and essentially all the milk fat at pH 4.5 and/or by adding chymosin. The whey fraction contains the albumin;
2. Affinity-chromatography of albumin on Cibacron blue 3GA-Sepharose CL-6B (Harvey (1980) in: Methods of Plasma Protein Fractionation, op. cit.) This step serves both to remove proteins other than albumin and to decrease the volume to be handled about 30-fold. Albumin is eluted from this matrix with 0.15 M NaCl and 20 mM sodium salicylate at pH 7.5;
3. Buffer-exchange on Sephadex G-25: desalting into 0.025 M sodium acetate, adjustment to pH 5.2, followed by filtration;
4. Anion-exchange chromatography on DEAE-Sepharose CL-6B. Desorption of albumin at pH 4.5;
5. Cation-exchange chromatography on CM-Sepharose CL-6B. Albumin elution with 0.11 M sodium acetate, pH 5.5 and concentration of albumin at a 6% (w/v) solution by ultrafiltration; and
6. Gel filtration on Sephacryl S-200. Fraction of high-molecular weight protein (e.g. albumin polymers, pyrogens) is discarded. The main fraction (albumin monomers) is concentrated by ultrafiltration and formulated.

It is to be noted that steps 3-6 are essentially identical to the method described by Curling and others (Curling (1980) op. cit.; Curling et al. (1982) op. cit.; Berglöf et al. (1982) op. cit.) for the purification of hSA from plasma.

### EXAMPLE 12

### Alternate Construction of Transgenes Encoding hLF

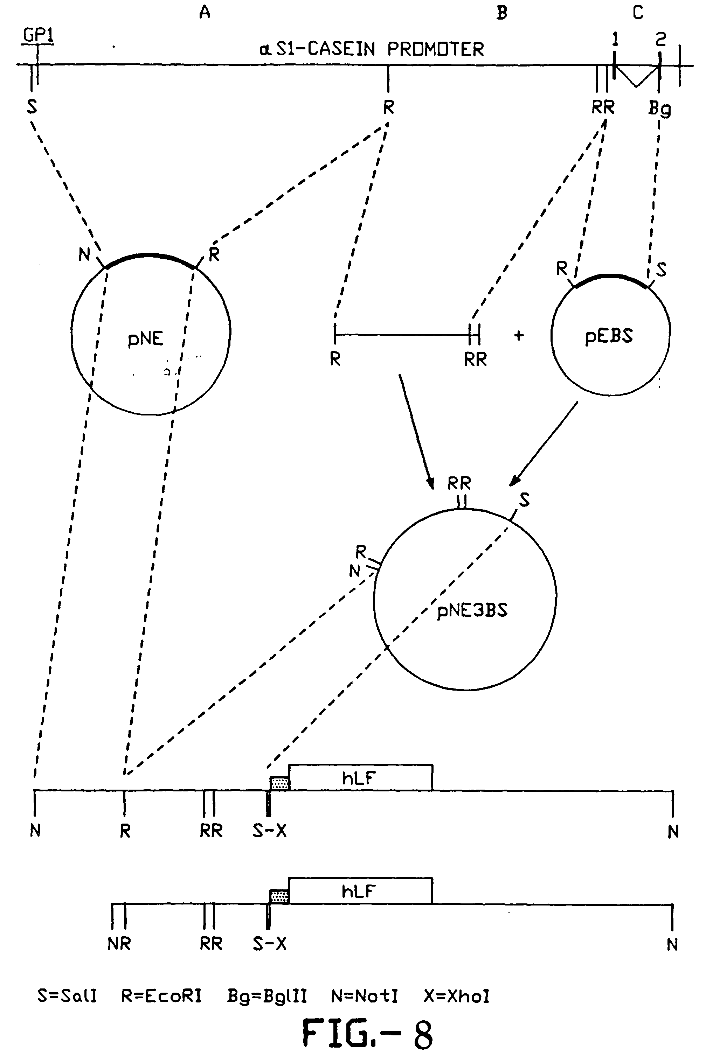

This example describes the construction of two hLF transgenes wherein the first contains approximately 16kb of αS1-casein 5' expression regulation sequence (pGP1hLF (16kb) also referred to as p16,8HLF4) and the second contains approximately 7.9kb of αS1-casein 5' expression regulation sequence (pGPlhLF (8kb) also referred to as p8.8HLF4). The overall strategy for these constructions is depicted in Fig. 8.

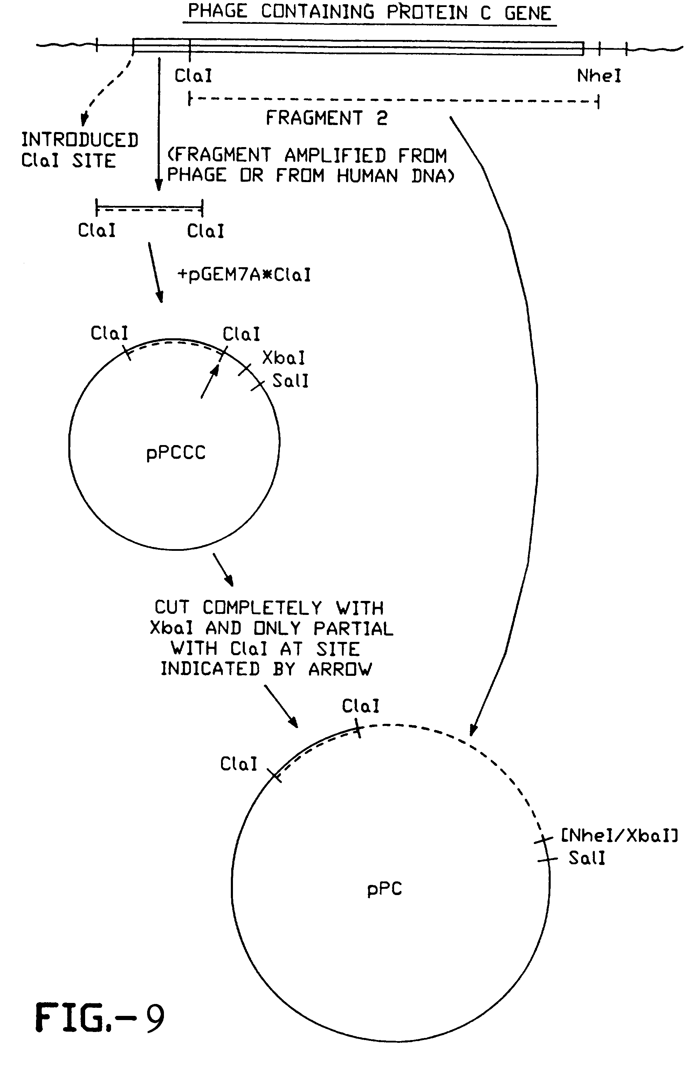

A 1.8kb EcoRI-BglII fragment (fragment C in Fig. 9) was isolated from phage clone GP1. This fragment runs from position -100 of the transcription start site into the second exon of the αS1-casein gene. The BglII site lies at the junction of the first entron and second exon of the αS1-casein gene. The 3' end containing the BglII site was ligated to a synthetic BglII-ClaI linker and subcloned into the plasmid pUC19. The resulting plasmid is designated pEBS.

Fragment B in Fig.8 was isolated as an EcoRI fragment and cloned into the EcoRI site of pEBS. Fragment B includes sequences from position -7500 to position -100 of the transcription start site in the αS1-casein gene. The plasmid so formed is designated pEB3S and contains the combination of fragments B and C is the 8.9kb EcoRI-ClaI fragment running from position -7500 to position +1400 of the transcription start site. The 8.9kb EcoRI-ClaI fragment from pEB3, obtained by complete digestion with ClaI and partial digestion with EcoRI was isolated and subcloned into EcoRI-ClaI cut pKUN2 ( a derivative of pKUN; Gene (1986) 46, 269-276 containing a NotI restriction site) to form pNE3BS.

An 8.5kb ClaI-EcoRI fragment (fragment A in Fig. 9) running from position -16000 to position -7500 of the transcription start site was isolated from phage GP1. It was thereafter subcloned into pUC19 to form pSE. Using synthetic oligonucleotide, a unique NotI site was introduced into the ClaI site thereby destroying it. The resulting plasmid is designated pNE.

The insert from pNE was isolated as a NotI-EcoRI fragment and together with the EcoRI-ClaI insert from pNE3BS was ligated into the cloning vector pKUN2. The resulting plasmid pGP1 (Δ2ex) contains 16kb of αS1-casein promoter plus the 5' end of the gene to the BglII site at the border of the second exon.

The final plasmid (16,8HLF4) containing the transgene was assembled using the NotI-ClaI fragment from clone pGPI (Δ2ex) and the Xho-NotI fragment from clone pHLF 3' 10kb. The structure of this transgene is the same as previously described herein.

As a minor modification to this plasmid the SalI site of this plasmid was removed by cutting with SalI and inserting a linker that contains a NotI site, but not a SalI site. Subsequently, a SalI site was introduced just downstream of the hLF sequence by cutting the KpnI site at that position adding the following linker:

5'-CGTCGACAGTAC-3' (Seq. ID No.: 18)
CATGGCAGCTGT-5' (Seq. ID No.: 19)

In effect, the hLF sequence is now surrounded by two unique restriction sites (ClaI and SalI) and can be replaced by any recombinant ANA sequence that has a ClaI-site at the 5'- end and a SalI-site at the 3'- end.

Another transgene was constructed that is identical to the foregoing except that it contains only about 8kb of 5' αS1-casein expression regulation sequence. It was constructed by taking the NotI-ClaI fragment from pNE3BS and fusing it directly into Xho-NotI fragment from clone pHLF 3' 10kb. The resulting plasmid was designated pGPIhLF (7kb) (also referred to as p8.8HLF4).

Plasmid 16,8hLF4 was modified to contain a hybrid splice signal (αS1-casein-IgG) described in examples 3 and 5. The resulting plasmid was designated 16,8hLF3 and is identical to 16,8hLF4 except for the presence of a hybrid intron versus a "natural" casein intron in the 5'-UTR.

The hLF signal sequence can also be used in all of the cDNA constructs disclosed herein instead of the casein signal sequence. This can be done in the following way: A synthetic oligo was made that contains the complete hLF signal sequence (see Fig. 2) plus a ClaI restriction site at the 5'-end and an EagI restriction site at the 3'-end. These restriction sites also border the casein-signal sequence in the other plasmids (e.g., p16,8hLF4). A fragment containing the hLF-cDNA surrounded by ClaI and SalI sites was cloned in pGEM7 (Stratagene, Inc.) containing a ClaI and SalI site. The resulting plasmid was digested with ClaI and EagI and used as a vector to accommodate the ClaI-EagI fragment containing the hLF sequence. From the positive clones, the cDNA, with its own sequence, was excised as a ClaI-SalI fragment and inserted in ClaI-SalI digested p16,8hLF4 to generate p16,8hLF5. Similarly, this Cla-Sal fragment containing the hLF-cDNA plus hLF signal sequence can be inserted in any hLF cDNA vector.

### EXAMPLE 13

### Construction of Transgene Cassette for Genomic Recombinant DNA

The plasmids described so far all contain regions derived from the bovine αS1-casein untranscribed regions (including intervening sequences). When a genomic gene is to be expressed that already contains untranslated regions and intervening sequences permissive for high expression, it is preferable to use expression cassettes where the flanking regions of the αS1-casein gene are operably linked to the untranslated regions of the gene to be expressed. Such an expression cassette is p-16kb,CS and was constructed as follows: plasmid pS1 3'5'hLF was used as a template in a PCR experiment. This plasmid contains 680 bp of promoter sequence of the αS1-casein gene as well as its first exon. the rest of this plasmid is not relevant for this experiment. The upstream primer was located just upstream of the insert in the plasmid moiety (just upstream of a NotI restriction site). Its sequence is: 5'-CGA CGT TGT AAA ACG ACGG-3'.

The downstream primer was located in exon 1. Its sequence matches the first 19 bp of the exon exactly and also has a non-hydridizing region of 17 bp containing a ClaI and a SalI site. It has the following sequence:

5'-ATTGTCGACTTATCGATGGGTTGATGATCAAGGTGA-3'

The amplified fragment was digested with NotI and SalI and ligated into pKUN2 (see Example 12 ). The resulting plasmid (p-680CS) therefore harbors a proximal promoter fragment from -680 to + 19, plus two restriction sites just downstream of those 19 bp.

This plasmid was digested with NotI (just upstream of - 680) and NsiI (at-280) and used as a vector to ligate to a fragment running from a NotI site (just upstream of -16kb) to NsiI (-280) isolated from p16,8hLF4 (Example 13). This plasmid (p-16kb,Cs) therefore harbors a promoter fragment from ≈-16,000 to +19. It can be used to insert genomic genes that carry their own UTR's and poly(A)-signal. After insertion of the genomic gene as a ClaI-SalI fragment, the αS1-casein 3'-flanking region can be inserted as a SalI-fragment.

### EXAMPLE 14

### Construction of Transgene for Production of Protein C

The genomic sequence of Protein C has been published. Foster, et al. (1985) Proc. Natl. Acad. Sci. USA 82, 4673-4677. This sequence, however, does not include the first exon which was identified through the cDNA sequence published by Beckman, et al. (1985) Nucl. Acids Res. 13, 5233-5247. The first exon of Protein C is located at position -1499 to -1448 in the Foster sequence. The transgene for expressing and secreting Protein C into the milk of bovine species is shown in Fig. 9. This transgene was constructed as follows.

A human genomic library in EMBL-3 (Clonotech) is probed with a sequence specific for protein C. A purified phage DNA prep containing the complete Protein C gene is isolated. The phage is isolated from an E. coli strain having the Dam phenotype, such a strain GM113. This results in cloned DNA which is not methylated and as such all ClaI restriction sites can be cleaved.

A ClaI NheI fragment running from positions +1333 to 11483 is isolated. This is designated fragment I.

pGEM7 (Stratogene, Inc.) is digested with SphI and SmaI. The region in between is replaced by the corresponding region of plasmid pKUN (Gene (1986) 46, 269-276). The resulting plasmid is designated pGEM7A and has the following restriction map in the relevant region:

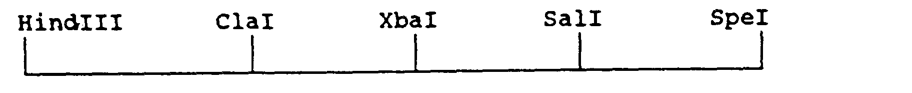
HindIII ClaI XbaI SalI SpeI

Two primers are synthesized. Primer GP125 has the following sequence:

5' - CAA ATC GAT TGA ACT TGC AGT ATC TCC ACG AC - 3'
ClaI

Primer GP 126 has the following sequence:

5' - GGG ATC GAT CAG ATT CTG TCC CCC AT - 3'
ClaI

Primer GP125 has an overlap with exon O (position 654 to 675 of the Protein C gene) and introduces a ClaI site in the 5' untranslated region. Exon O is the exon not identified by Foster, et al. Primer GP126 overlaps the region from 1344 to 1315 in the Protein C gene. This region contains a ClaI site.

The region between position 654 and 1344 is amplified using either human DNA or phage DNA as a template. The so amplified material is digested with ClaI and cloned in vector pGEN7a to form pPCCC. This vector is propagated in a dam negative strain such as GM113 and partially cut with ClaI (only the plasmids that are cut once with ClaI at position 1340 are of interest) and completely with XbaI. The ClaI NheI fragment (fragment 1) is cloned into this vector. The resultant plasmid is designated pPC. Its structure is shown in Fig. 9. From this plasmid, the Protein C transgene is isolated as a ClaI-SalI fragment and ligated into pl6Kb, CS (See Example 13) to generate a transgene capable of expressing Protein C in bovine milk, this plasmid is designated p16 Kb,CS,PC.

The transgene contained within plasmid p 16 Kb, CS, PC is excised with NotI and used to generate transgenic bovine species as previously described. Such transgenic animals are capable of producing protein C in their milk.

All references disclosed herein are expressly incorporated by reference.

## Claims

1. The use of in vitro maturation of a bovine oocyte in the production of a transgenic bovine species of desired phenotype.

2. A method for producing an embryo of a transgenic bovine species which is capable of producing a recombinant polypeptide in at least one cell type, comprising:
obtaining an oocyte from bovine ovaries;
obtaining maturation of the oocyte in vitro and fertilizing to form a zygote;
introducing, optionally by microinjection, a transgene into the zygote; and
forming an embryo from the zygote.

3. A method of producing a transgenic bovine species comprising transplanting an embryo which has been produced by a method as defined in claim 2 into a recipient female bovine parent to permit the embryo to gestate and said parent to give birth.

4. A method of claim 2 or claim 3, wherein the transgene encodes a polypeptide which is produced in the milk of said transgenic species.

5. A method of claim 4, wherein said recombinant polypeptide is a heterologous polypeptide, optionally a human milk protein, a human serum protein, or an industrial enzyme.

6. A method of claim 5, wherein said heterologous polypeptide is a human secretory or other immunoglobulin, human lysozyme, human lactoferrin, human lactoglobulin, human α-lactalbumin, human bile salt-stimulated lipase, human serum albumin, human Factor VIII, human Factor IX, human Protein C, a protease, a lipase, a chitinases, or a ligninase.

7. A method of any one of claims 2 to 6 also comprising the steps of:
(a) methylating said transgene;
(b) introducing said methylated transgene into said fertilized oocyte to permit integration of said transgene into the genomic DNA of said fertilized oocyte;
(c) culturing the individual ovum formed thereby to give a pre-implantation embryo thereby replicating the genome of said fertilized ovum;
(d) removing at least one cell from said pre-implantation embryo and lysing said at least one cell to release the DNA contained therein;
(e) contacting said released DNA with a restriction endonuclease capable of cleaving said methylated transgene but incapable of cleaving the unmethylated form of said transgene formed after integration into an replication of said genomic DNA; and
(f) detecting whether said cells from said pre-implantation embryo contain a transgene which is resistant to cleavage by said restriction endonuclease as an indication of whether said pre-implantation embryo has integrated said transgene.

8. A method of any one of claims 2 to 7, wherein said transgene comprises a recombinant intervening sequence.

9. A process for producing bovine milk containing a recombinant polypeptide, which comprises:
obtaining an oocyte from bovine ovaries;
obtaining maturation of the oocyte in vitro and fertilizing the oocyte to form a zygote;
introducing, optionally by microinjection, a transgene into the zygote;
forming an embryo from the zygote;
transplanting said embryo into a recipient female bovine parent to permit the embryo to gestate and said parent to give birth; and
obtaining milk from said transgenic bovine species.

10. A process for producing bovine milk containing a recombinant polypeptide, which comprises obtaining milk from a transgenic bovine species which has been produced by a method of any one of claims 3 to 8 or by a use of claim 1.

11. A process for producing a recombinant polypeptide, which comprises:
obtaining an oocyte from bovine ovaries;
obtaining maturation of the oocyte in vitro and fertilizing the oocyte to form a zygote;
introducing, optionally by microinjection, a transgene into the zygote;
forming an embryo from the zygote;
transplanting said embryo into a recipient female bovine parent to permit the embryo to gestate and said parent to give birth;
obtaining milk from said transgenic bovine species; and
recovering said recombinant polypeptide from said milk.

12. A process for producing a recombinant polypeptide, which comprises recovering said polypeptide from milk which has been produced by a process of claim 9 or claim 10.

13. A process for producing a pharmaceutical, which comprises:
obtaining an oocyte from bovine ovaries;
obtaining maturation of the oocyte in vitro and fertilizing the oocyte to form a zygote;
introducing, optionally by microinjection, a transgene into the zygote;
forming an embryo from the zygote;
transplanting said embryo into a recipient female bovine parent to permit the embryo to gestate and said parent to give birth;
obtaining milk from said transgenic bovine species;
recovering said recombinant polypeptide from said milk; and
using said recombinant polypeptide in making a pharmaceutical.

14. A process for producing a pharmaceutical, which comprises using a recombinant polypeptide which has been produced by a process of claim 11 or claim 12 to make a pharmaceutical.

## Patentansprüche

1. Die Verwendung der *in vitro*-Reifung einer Rinder-Oozyte zur Herstellung einer transgenen Rinderart des gewünschten Phenotyps.

2. Verfahren zur Herstellung eines Embryos einer transgenen Rinderart, die zur Erzeugung eines rekombinanten Polypeptids in mindestens einem Zelltyp fähig ist, umfassend
Isolieren einer Oozyte aus Rinderovarien,
Reifung der Oozyte *in vitro,* und Befruchtung unter Bildung einer Zygote,
Einschleusung eines Transgens in die Zygote, gegebenenfalls durch Mikroinjektion, und
Bildung eines Embryos aus der Zygote.

3. Verfahren zur Herstellung einer transgenen Rinderart, umfassend das Transplantieren eines nach Anspruch 2 hergestellten Embryos zum Heranreifen des Embryo und Gebähren der Leihmutter.

4. Verfahren nach Anspruch 2 oder 3, in dem das Transgen für ein Polypeptid kodiert. das in der Milch der transgenen Art gebildet wird.

5. Verfahren nach Anspruch 4, in dem das rekombinante Polypeptid ein heterologes Polypeptid ist, gegebenenfalls ein menschliches Milchprotein, ein menschliches Serumprotein oder ein technisches Enzym.

6. Verfahren nach Anspruch 5, in dem das heterologe Polypeptid ein menschliches sekretorisches oder anderes Immunoglobulin, menschliches Lysozym, menschliches Lactoferrin, menschliches Lactoglobulin, menschliches α-Lactalbumin, menschliche durch Gallensalz-stimulierte Lipase, menschliches Serumalbumin, menschlicher Faktor 8, menschlicher Faktor 9, menschliches Protein C, eine Protease, eine Lipase, eine Chitinase oder eine Ligninase ist.

7. Verfahren nach einem der Ansprüche 2 bis 6, weiterhin umfassend die Schritte:
a) Methylierung des Transgens,
b) Einschleusung des methylierten Transgens in die befruchtete Oozyte zur Integration des Transgens in die genomische DNA der befruchteten Oozyte,
c) Kultivierung der einzelnen hierdurch gebildeten Eizelle zu einem PreimplantationsEmbryo, dadurch Replikation des Genoms der befruchteten Eizelle,
d) Entfernen mindestens einer Zelle des Preimplantationsembryos und Lysieren mindestens einer Zelle unter Freisetzen der enthaltenen DNA,
e) In-Kontakt-Bringen der freigesetzten DNA mit einer Restriktionsendonuclease, die zur Spaltung des methylierten Transgens fähig ist, aber unfähig zur Spaltung der nicht-methylierten Form des Transgens ist, die nach Integration und Replikation des Genoms gebildet wird, und
f) Nachweis, ob die Zellen des Preimplantationsembryos ein Transgen enthalten, das von der Restriktionsendonuclease nicht gespalten wird, als ein Indikator, ob das Preimplantationsembryo das Transgen integriert hat.

8. Verfahren nach einem der Ansprüche 2 bis 7, in dem das Transgen eine rekombinante Intron-Sequenz umfaßt.

9. Verfahren zur Herstellung von Rindermilch enthaltend ein rekombinantes Polypeptid, umfassend:
Isolieren einer Oozyte aus Rinderovarien,
Reifung der Oozyte *in vitro,* und
Befruchtung der Oozyte unter Bildung einer Zygote,
Einschleusung eines Transgens in die Zygote, gegebenenfalls durch Mikroinjektion,
Bildung eines Embryos aus der Zygote,
Transplantieren des Embryos in eine Rinderleihmutter zum Heranreifen des Embryos und Gebären der Leihmutter, und
Gewinnen der Milch aus der transgenen Rinderart.

10. Verfahren zur Herstellung von Rindermilch enthaltend ein rekombinantes Polypeptid, umfassend Isolieren der Milch von einer transgenen Rinderart, die nach einem Verfahren eines der Ansprüche 3 bis 8 oder durch eine Verwendung gemäß Anspruch 1 erhalten wird.

11. Verfahren zur Herstellung eines rekombinanten Polypeptids, das umfaßt:
Erhalten einer Oozyte aus Rinderovarien
Reifung der Oozyte *in vitro,* und
Befruchtung der Oozyte unter Bildung einer Zygote,
Einschleusung eines Transgens in die Zygote, gegebenenfalls durch Mikroinjektion,
Bildung eines Embryos aus der Zygote,
Transplantieren des Embryos in eine Rinderleihmutter zum Heranreifen des Embryos und Gebären der Leihmutter,
Gewinnen der Milch aus der transgenen Rinderart, und
Zurückgewinnen des rekombinanten Polypeptids aus der Milch.

12. Verfahren zur Herstellung eines rekombinanten Polypeptids, das umfaßt Zurückgewinnen des Polypeptids aus Milch, die durch ein Verfahren nach Anspruch 9 oder 10 hergestellt wurde.

13. Verfahren zur Herstellung eines Arzneimittels, das umfaßt:
Erhalten einer Oozyte aus Rinderovarien
Reifung der Oozyte *in vitro,* und
Befruchtung der Oozyte unter Bildung einer Zygote,
Einschleusung eines Transgens in die Zygote, gegebenenfalls durch Mikroinjektion,
Bildung eines Embryos aus der Zygote,
Transplantieren des Embryos in eine Rinderleihmutter zum Heranreifen des Embryos und Gebären der Leihmutter,
Gewinnen der Milch aus der transgenen Rinderart,
Zurückgewinnen des rekombinanten Polypeptids aus der Milch, und
Verwenden des rekombinanten Polypeptids bei der Herstellung eines Arzneimittels.

14. Verfahren zur Herstellung eines Arzneimittels, umfassend das Verwenden eines rekombinanten Polypeptids, das durch ein Verfahren nach Anspruch 11 oder 12 zur Herstellung eines Arzneimittels hergestellt wurde.

## Revendications

1. Utilisation de la maturation in vitro d'un ovule bovin pour la production d'une espèce bovine transgénique d'un phénotype désiré.

2. Méthode pour produire un embryon d'une espèce bovine transgénique capable de produire un polypeptide recombinant dans au moins un type cellulaire comprenant :
l'obtention d'un ovule à partir d'ovaires bovins ;
l'obtention de la maturation de l'ovule in vitro et sa fécondation pour former un zygote ;
l'introduction éventuellement par micro-injection, d'un transgène dans le zygote ; et
la formation d'un embryon à partir du zygote.

3. Méthode de production d'une espèce bovine transgénique comprenant la transplantation d'un embryon qui a été produit par une méthode telle que définie dans la revendication 2 chez une femelle bovine parente receveuse pour permettre la gestation de l'embryon et permettre à ladite parente de mettre bas.

4. Méthode selon la revendication 2 ou 3, où le transgène code pour un polypeptide qui est produit dans le lait de ladite espèce transgénique.

5. Méthode selon la revendication 4, où ledit polypeptide recombinant est un polypeptide hétérologue éventuellement une protéine du lait humain, une protéine du sérum humain, ou une enzyme industrielle.

6. Méthode selon la revendication 5, où ledit polypeptide hétérologue est une immunoglobuline humaine sécrétoire ou autre, le lysozyme humain, la lactoferrine humaine, la lactoglobuline humaine, l'α-lactalbumine humaine, la lipase stimulée par les sels biliaires, la sérum albumine humaine, le facteur VIII humain, le facteur IX humain, la protéine C humaine, une protéase, une lipase, une chitinase, ou une ligninase.

7. Méthode selon l'une quelconque des revendications 2 à 6 comprenant en outre les étapes consistant à :
(a) faire une méthylation dudit transgène ;
(b) introduire ledit transgène méthylé dans lesdits ovules fécondés pour permettre l'intégration dudit transgène dans l'ADN génomique desdits ovules fécondés ;
(c) cultiver les ovules individuels ainsi constitués jusqu'à former des embryons préimplantables par la réplication du génome de chacun desdits ovules fécondés ;
(d) prélever au moins une cellule de chacun desdits embryons préimplantables et lyser ladite cellule pour libérer l'ADN contenu à l'intérieur ;
(e) mettre en contact ledit ADN libéré avec une endonucléase de restriction capable de cliver ledit transgène méthylé mais incapable de cliver la forme non méthylée dudit transgène formée après son intégration et la réplication dudit ADN génomique ; et
(f) détecter parmi les cellules provenant desdits embryons préimplantables quelles sont celles qui contiennent un transgène résistant au clivage par ladite endonucléase de restriction, ces cellules étant une indication des embryons préimplantables qui ont intégré ledit transgène.

8. Méthode selon l'une quelconque des revendications 2 à 7, où ledit transgène comprend une séquence intercalaire recombinante.

9. procédé pour la production de lait de bovin contenant un polypeptide recombinant, qui comprend :
l'obtention d'un oocyte à partir d'ovaires bovins ;
l'obtention de la maturation de l'oocyte in vitro et la fécondation de l'oocyte pour former un zygote ;
l'introduction, éventuellement par micro-injection, d'un transgène dans le zygote ;
la formation d'un embryon à partir du zygote ;
la transplantation dudit embryon dans une femelle bovine parente receveuse pour permettre la gestation de l'embryon et permettre à ladite parente de mettre bas ; et
l'obtention du lait à partir de ladite espèce bovine transgénique.

10. Procédé pour la production de lait bovin contenant un polypeptide recombinant, qui comprend l'obtention du lait à partir d'une espèce bovine transgénique qui a été produite par une méthode selon l'une quelconque des revendications 3 à 8 ou par une utilisation selon la revendication 1.

11. Procédé pour la production d'un polypeptide recombinant qui comprend :
l'obtention d'un oocyte à partir d'ovaires bovins ;
l'obtention de la maturation de l'oocyte in vitro et la fécondation de l'oocyte pour former un zygote ;
l'introduction, éventuellement par micro-injection, d'un transgène dans le zygote ;
la formation d'un embryon à partir du zygote ;
la transplantation dudit embryon dans une femelle bovine parente receveuse pour permettre la gestation de l'embryon et permettre à ladite parente de mettre bas ;
l'obtention du lait à partir de l'espèce bovine transgénique ; et
la récupération dudit polypeptique recombinant de ce lait.

12. Procédé pour la production d'un polypeptide recombinant qui comprend la récupération dudit polypeptide à partir du lait qui a été produit par un procédé selon la revendication 9 ou 10.

13. Procédé pour la production d'un produit phamaceutique, qui comprend :
l'obtention d'un oocyte à partir d'ovaires bovins ;
l'obtention de la maturation d'un oocyte in vitro et la fécondation de l'oocyte pour former un zygote ;
l'introduction, éventuellement par micro-injection, d'un transgène dans le zygote ;
la formation d'un embryon à partir du zygote ;
la transplantation dudit embryon dans une femelle bovine parente receveuse pour permettre la gestation de l'embryon et permettre à ladite parente de mettre bas ;
l'obtention du lait à partir de ladite espèce bovine transgénique ;
la récupération dudit polypeptide recombinant de ce lait ; et
l'utilisation dudit polypeptide recombinant pour la fabrication d'un produit pharmaceutique.

14. Procédé de préparation d'un produit pharmaceutique, qui comprend l'utilisation d'un polypeptide recombinant qui a été produit par un procédé selon la revendication 11 ou 12 pour fabriquer un produit pharmaceutique.
